# EUROPEAN PATENT SPECIFICATION

(11) **EP 1 711 104 B1**
(45) Date of publication and mention of the grant of the patent: **12.03.2014**
(21) Application number: 05700068.9
(22) Date of filing: 14.01.2005
(51) Int. Cl.: A61B 5/0472, A61B 5/0402

(54) **METHOD AND APPARATUS FOR ECG-DERIVED SLEEP DISORDERED BREATHING MONITORING, DETECTION AND CLASSIFICATION**
VERFAHREN UND GERÄT FÜR DIE EKG-ÜBERWACHUNG VON ATMUNGSSTÖRUNGEN IM SCHLAF, NACHWEIS UND KLASSIFIKATION
PROCEDE ET DISPOSITIF PERMETTANT DE SURVEILLER, DE DETECTER ET DE CLASSIFIER DES TROUBLES RESPIRATOIRES DU SOMMEIL A PARTIR D'UN ELECTROCARDIOGRAMME

(30) Priority: 16.01.2004 AU 2004900177
(43) Date of publication of application: 18.10.2006
(73) Proprietor: Compumedics Limited, Abbotsford, Victoria 3067 (AU)
(72) Inventor: BURTON, David, Camberwell, Victoria 3124 (AU)
(74) Representative: Hands, Lewis Roger
(86) International application number: PCT/AU2005/000036
(87) International publication number: WO 2005/067790

(56) References cited:
- EP-A- 1 645 306
- WO-A-01/91631
- US-A- 5 458 137
- US-A- 5 769 084
- US-B1- 6 411 843
- US-B1- 6 415 174
- ZYWIETZ C ET AL: "Polysomnographic sleep recording with simultaneously acquired 12 lead ecgs: a study for detection and validation of apnea related ecg changes" COMPUTERS IN CARDIOLOGY 2002. MEMPHIS, TN, SEPT. 22 - 25, 2002; [COMPUTERS IN CARDIOLOGY], NEW YORK, NY : IEEE, US, vol. VOL. 29, 22 September 2002 (2002-09-22), pages 573-576, XP010624217 ISBN: 978-0-7803-7735-6
- MAZZANTI B ET AL: "Validation of an ECG-derived respiration monitoring method" COMPUTERS IN CARDIOLOGY 2003. THESSALONIKI, GREECE, SEPT. 21 - 24, 2003; [COMPUTERS IN CARDIOLOGY], NEW YORK, NY : IEEE, US, vol. VOL. 30, 21 September 2003 (2003-09-21), pages 613-616, XP010698979 ISBN: 978-0-7803-8170-4
- NAZERAN ET AL: 'Computer-Based Analysis of Heart Rate Variability Signal for Detection of Sleep Disordered Breathing in Children.' PROCEEDINGS OF THE 25TH ANNUAL INTERNATIONAL CONFERENCE ON IEEE EBMS. 17 September 2003 - 21 September 2003, pages 338 - 341, XP010693205
- VIJENDRA ET AL: 'Frequency Domain Analysis of Heart Rate Variability in Sleep Disordered Breathing.' PROCEEDINGS OF THE 25TH ANNUAL INTERNATIONAL CONFERENCE ON IEEE EBMS. 17 September 2003 - 21 September 2003, pages 208 - 211, XP010693137
- KISTAS ET AL: 'Short-Term Analysis of Heart Rate Variability Using Wavelet Packets: An Efficient Detector of Sleep Apnea Episodes.' PROCEEDINGS OF THE 2ND JOINT EMBS/BEMS CONFERENCE. 23 October 2002 - 26 October 2002, pages 88 - 89, XP010621222
- CHAZAL ET AL: 'Automated Processing of the Single Lead SG for the Detection of Obstructive Sleep Apnea.' IEEE TRANSACTIONS ON BIOMEDICAL ENGINEERING. vol. 50, no. 6, 06 June 2003, pages 686 - 696, XP011097424
- NAZERAN ET AL: 'A User-Friendly Integrated Software Environment for Heart Rate Variability Analysis of Sleep Disordered Breathing.' PROCEEDINGS OF THE 25TH ANNUAL INTERNATIONAL CONFERENCE ON IEEE EBMS. 17 September 2003 - 21 September 2003, XP010691299
- PENZET ET AL: 'Systematic Comparison of Different Algorithms for Apnoea Detection Based on Electrocardiogram Recordings.' MEDICAL AND BIOLOGICAL ENGINEERING & COMPUTING. vol. 40, 2002, pages 402 - 407, XP001185110
- ROCHE ET AL: 'Predicting Sleep Apnoea Syndrome from Heart Period: A Time Frecuency Wavelet Analysis.' EUROPEAN RESPIRATORY JOURNAL. vol. 22, 2003, pages 937 - 942, XP008110795
- ADACHI ET AL: 'Clinical Significance Of Pulse Rate Rise Sleep As A Screening Marker For The Assessment Of Sleep Fragmentation In Sleep-Disordered Breathing.' SLEEP MEDICINE. vol. 4, 2003, pages 537 - 542, XP008110798

## Description

### Field of Invention

The present invention relates generally to sleep disordered breathing (SDB) and monitoring and analysis of electro-cardiology. In particular the invention relates to analysis of a subject's respiration effort as derived from electro-cardiographic measurements. The analysis may include a capability to distinguish and classify in real-time or breath-by-breath or post signal acquisition of SDB data. The SDB may be classified into apnea, hypopnoea, shallow breathing, Cheyne-Stokes respiration (CSR), Central Sleep apnea (CSA), Obstructive Sleep apnea (OSA), Mixed Sleep apnea (MSA), the latter being a combination of CSA and OSA, body movement, arousal, artifact, respiratory event related arousal (RERA), therapeutic event related arousal (TERA) and unclassified SDB.

The present invention may include real-time ambulatory holter monitoring incorporating a capability to derive and display thoracic and abdominal ECG derived respiration traces and phase relationships, together with verification of electrode placement and guidance to achieve a preferred connection.

The monitoring and analysis capabilities of the present invention may be applied to treatment countermeasures including continuous positive air pressure (CPAP), automatic positive air pressure (APAP), pacemaker, ventilation, oxygen treatment and drug administration.

### Background

Around 10% of population in the Western World is affected by 84 varieties of sleep disorders. Linkages between SDB and cardiovascular disease are becoming rapidly evident. In the USA alone, over 20 million people suffer sleep apnea, while there are over 4 million people diagnosed with Congestive Heart Failure (CHF), with an alarming 15 million more at risk of developing this condition. Significantly, recent research has shown that of those with CHF, around 50% have some form of breathing related sleep disorder. Given that two separate areas of medicine traditionally treat these two conditions, namely cardiovascular and respiratory medicine respectively, little has been done so far to simultaneously monitor, analyse and treat the two conditions.

Sleep-Disordered Breathing (SDB) has been associated with increases in a subject's daytime sympathetic activity. Although the reasons as to why these subjects have increases in sympathetic drive is not known, it has been reported that they have faster heart rates, decreased heart rate variability and increased blood pressure variability, where these symptoms are associated with an increased risk for hypertension and cardiac damage. Arrhythmias have been reported to be associated with SDB. Bradyarrhythmias have been noted as possibly being a consequence of apneic events.

Two major types of SDB have been associated with heart failure and these are obstructive sleep apnea (OSA) and central sleep apnea (CSA). CSA is characterised by a periodic cessation of breathing effort while OSA is characterised by occlusion of the upper airway due to airway collapse, despite continued effort to breathe.

Central apnea has been commonly reported in patients with heart failure. Although the mechanisms for central apnea in heart failure patients is not well understood, it is known that sympathetic activity and levels of norepinephrine are higher in heart failure patients with central apnea than those heart failure patients without central apnea.

Somers VK (2002) noted that although it is unknown why heart failure patients have such a high prevalence of CSA, possibilities include prolonged circulation time, abnormalities in respiratory control, and increased sensitivity to CO₂. It was further noted that heart failure patients have an increased chemoflex response to hypocapnia and those patients with the greatest sensitivity to CO₂ are those most likely to have CSA. It has been reported that intercardiac filling pressures may also play a role in the genesis of CSA. Heart failure patients with CSA were reported to have higher pulmonary capillary wedge pressure measurements and lower arterial CO₂ levels than those heart failure patients without CSA.

It has also been reported that the presence of central sleep apnea in patients with heart failure appears to be an independent predictor of increased mortality.

Sin DD et al., (2000) reported that treatment of sleep apnea using carefully titrated positive air pressure treatment may improve transplant free survival in heart failure patients. It was also noted that although positive air pressure improved cardiac function in patients with CSR-CSA, the same was not apparent in patients without CSR-CSA. Treatment of CSA includes theophylline, low levels of nasal oxygen or CO₂, and more recently CPAP.

Somers VK (2002) summarised his comprehensive review of "Mechanisms Linking Sleep to Cardiovascular Death and Disease", by noting the compelling reasons supporting implications of sleep-related changes (particularly REM sleep) on blood pressure and subsequently may be associated with cardiac ischemia, vasospasm, or arrhythmia. It was also noted that SDB such as OSA and central apnea may also be important in pathophysiology of hypertension and heart failure.

Somers VK (2002) noted that prevalence of OSA in a systolic heart failure population has been estimated between 5 % and 10%, with prevalence as high as 50 % with patients with diastolic dysfunction. Somers further noted that OSA should be considered in patients with heart failure, particularly those who are obese and refractory to standard treatment.

Nieto, Young et al. (2000) in the largest cross-sectional study to date analysed participants in the Sleep Heart Health Study (a community-based multicenter study of 6132 participants aged > 40 years, and concluded that SDB is associated with systemic hypertension in middle-aged and older individuals of different sexes and different ethnic backgrounds.

A study conducted in Spain by Parra, Arboix, etal. (2000) investigated prevalence and behaviour of sleep-related breathing disorders (SRBD) associated with first-ever stroke or transient ischemia attack (TIA) by prospectively studying 161 consecutive patients admitted to their stroke unit and within 48-72 hours after admission (acute phase) instigated a portable respiratory recording. It was found that 71.4 % of patients had a hypopnea-hypopnea index (AHI) > 10 and 28 % had an AHI > 30, demonstrating that prevalence of SRBD with first-ever stroke or TIA expected from epidemiological data.

A similar study conducted in Germany involved 147 consecutive patients admitted to a neurological Rehabilitation department for a first-ever stroke, and showed similar results whereupon 61% of patients had an hypopnea-hypopnea index (AHI) < 5, 44 % had an RDI index of < 10, 32 % had an RDI index of < 15, 22 % had an RDI index of < 20, concluding high prevalence of SDB amongst stroke patients and recommending stroke examination include screening for SDB.

Cheynes-Stoke breathing (CSB) is documented as being an abnormal cyclical pattern of respiratory fluctuations observed during sleep in congestive heart failure (CHF) with poor prognosis.

Kales D (1999) noted that with 20 million Americans suffering from sleep hypopnea, studies indicated that 50% of the CHF population of 4 million Americans have sleep-disordered breathing and a third of these OSA-CHF sufferers also experience Cheyne-Stokes respiration (CSR).

Tateishi O., et al (2002) simultaneously monitored ambulatory electrocardiograms and respiration in 86 heart disease patients concluded that Heart Rate Variability can act as an indicator of presence of CSB in CHF patients, thereby enabling HRV to be used in outpatient conditions to identify CHF patients with poor prognosis.

Fletcher, BeBehnke, et al. (1985) reported that daytime systemic hypertension is seen in up to 90% of patients with sleep apnea syndrome. It was further reported by Fletcher in a study of 46 middle and older-aged men with "essential hypertension" that sleep apnea is associated with systematic hypertension in up to 30% of middle- and older-aged hypertensive men.

### Description of the Prior Art

Studies have indicated that presence of SDB in patients can contribute to symptoms of heart failure. For example, studies have shown that some Arrhythmias and Brady arrhythmias are linked to SDB. Nocturnal oxygen desaturation has also been linked to daytime hypertension. In fact, studies indicate that around 50% of congestive heart failure sufferers have some form of sleep-disordered breathing. As such, there is a need to study and take into account effects of SDB on patients suffering from cardiovascular disease.

Two major types of SDB, OSA and CSA, have been closely linked with heart failure and cardiovascular disease. CSA is typically characterized by a periodic cessation of breathing effort during sleep and OSA is characterized by occlusion of the upper airway during sleep due to airway collapse. Typically, these diseases are not monitored by most cardiologists because monitoring and treatment of SDB typically requires use of specialized pulmonary equipment such as respiration monitors and CPAP devices. However, studies have shown that presence of these ODB results in an increased mortality rate for patients who suffer heart failure.

Use of holter monitoring is a known method for detecting of cardiovascular disease in patients. This process typically involves connecting a patient to a holter recorder unit worn by the patient for a predetermined period of time, typically 24 hours. During this period of time, the patient's ECG is recorded by the holter recorder unit, and after the study is done, a cardiologist is able to download recorded ECG signals for the period and perform an analysis of the ECG during the entire period of time. However, despite a growing awareness of linkage between cardiovascular disease and sleep disordered breathing (SDB), the ECG signals are only typically studied to determine cardiovascular disease. Consequently, devices which measure patient's ECG, such as holter recorders, have not typically been used for detecting and monitoring of SDB. As such, a significant benefit can be achieved by using cardiac studies, such as holter monitoring, to also detect SDB.

Current holter ECG analysis methods are vulnerable because they can fail to associate arrhythmia to the underlying respiratory disturbance causation. Inability to reliably detect CSR from ECG-derived respiration signals can hinder diagnostic outcomes, prognosis of such outcomes and appropriate treatment countermeasures.

A study investigating whether morthological ECG parameters are changed during sleep disorders, in particular during apnea is described in ZYWIETZ C ET AL: "Polysomnographic sleep recording with simultaneously acquired 12 lead ecgs: a study for detection and validation of apnea related ecg changes" COMPUTERS IN CARDIOLOGY 2002. MEMPHIS, TN, SEPT 22 - 25, 2002; [COMPUTERS IN CARDIOLOGY]' NEW YORK, NY: IEEE, US, vol. VOL.29, 22 September 2002 (2002-09-22), pages 573-576, XP010624217 ISBN: 978-0-7803-7735-6.

A method of taking and analysing ECG-derived respiration measurements is described in MAZZANTI B ET AL: "Validation of an ECG-derived respiration monitoring method" COMPUTERS IN CARDSIOLOGY 2003. THESSALONIKI, GREECE, SEPT. 21-24, 2003; [COMPUTERS IN CARDIOLOGY], NEW YORK, NY: IEEE, US, vol.VOL.30 - 21 September 2003 (2003-09-21), pages 613-616, XP010698979 ISBN: 978-0-7803-8170-4.

The discussion of the background to the invention herein is included to explain the context of the invention. This is not to be taken as an admission that any of the material referred to was published, known or part of the common general knowledge in Australia as at the priority date of any of the claims.

There is a need for a method and apparatus that can utilize a patient's ECG to determine existence of SDH or SDB in combination with cardiac events and/or heart rate variability (HRV).

According to the present invention, there is provided a method or processing an electrocardiogram (ECG) signal as defined in claim 1 hereinafter.

Further according to the present invention, there is provided an apparatus for detecting a physiological event as defined in claim 10 hereinafter.

The ECG signal recorded from the surface of a subject's chest is influenced by both motion of chest electrodes in relation to the heart, and changes in electrical impedance of the thoracic cavity. Movement of the chest in response to a subject's inspiration and expiration results in motion of the chest electrodes. Cyclic changes in thoracic impedance reflect filling and emptying of the lungs. This phenomenon gives rise to dynamic changes in impedance across the chest cavity and forms a basis of impedance plethysmography. The changes in impedance give rise to voltage or conductivity changes associated with ECG signal source generation, the latter being associated with changes in respiration or physiology.

In contrast to the prior art the system of the present invention may include placement of the electrical axis of the ECG electrode to maximise ECG signal strength and signal to noise ratio. The placement may optimise variation in electrical impedance between electrodes and corresponding variations in ECG signal with changes in thoracic and abdominal breathing movements.

Where only one ECG lead is available QRS area measurements from the lead may be used to derive a subject's respiration. Furthermore where only one ECG lead is available, signal to noise ratio may be enhanced when the lead axis is orthogonal to mean electrical axis.

The system of the present invention may distinguish CSA and OSA by utilising ECG derived detection of EMG breathing effort as evident during OSA versus CSA, and out of phase signals reflecting thoracic and abdominal effort during OSA versus no abdominal and thoracic effort during CSA.

Furthermore the system may enable real-time ECG derived respiration with separate thoracic and abdominal breathing effort monitoring, along with phase monitoring, display and measurement. The system may also enable guidance and validation for optimal electrode placement to ensure that changes in electrical signal axis and thoracic and abdominal movement ECG signal are evident and optimised.

The system may include three or more ECG electrodes attached to a subject in predetermined locations. Separate pairs of ECG electrodes are preferably positioned such that movement attributable to abdominal breathing and thoracic breathing may be separately measured and distinguished. The ECG electrodes may be positioned in two planes or in an orthogonal arrangement whereby improved signal to noise ratio may be achieved for accurate QRS analysis. The system may also include a capability to verify optimal placement of the electrodes.

This may enable predetermined electrode placement based on a balance between achieving optimal ECG QRS electrical signal to noise ratio using orthogonal electrode positioning, while at the same time allowing positioning of electrodes such that the ECG signal recorded from the surface of a subject's chest are influenced by both motion of the electrodes in relation to the heart, and changes in electrical impedance due to both abdominal and thoracic breathing movements. During OSA a subject exhibits breathing effort that can typically be detected as anti-phase breathing effort of the subjects abdomen and chest.

The system may include means to characterize ECG or HRV into respiratory and cardiac related constituents to provide more sensitive and precise measures of cardiac and respiratory function, including derivation of real-time measures of LFnu (normalized low-frequency power), and LF/HF ratio (low-frequency/high-frequency ratio), as a measure of sympathovagal balance or as a marker of illness severity.

The system may include means for monitoring and analyzing real-time or post data acquisition ECG signals including any combination of:
a) means for preoperative or other ICU application enabling monitoring and comparison of predetermined safe or predesignated operating ranges or thresholds, whereupon exceeding or operating outside these means can alter a local or remote user or healthcare worker or influence therapeutic treatment such as CPAP, APAP, oxygen concentration, drug perfusion or delivery, ventilation, or pacemakers;
b) means to detect HRV when a likelihood or probability of atrial fibrillation (AF) or onset of same is detected;
c) means to predict or avoid onset or excessive risk of AF with early detection of suspicious HRV symptoms where such means can awaken a sleeping patient and/or modify APAP, CPAP, ventilation or oxygen treatment administration to an individual, where treatment modification can include, for example reduction of APAP or CPAP or oxygen or ventilation gas administration;
d) means to compare ECG or HRV measures to a global database of normal and abnormal values, threshold and ranges enabling detection of incidence of, or onset, or prediction or onset of, elevated risk or physiological stress relating to illness or a deterioration in state of health;
e) means to alert or alarm a subject being monitored, or remote health worker, of onset or prediction of onset of cardiac risk or a need or determination of optimal countermeasure treatment, such as control of APAP, ventilation, pacemaker or administration of gas or drugs (such as oxygen or anesthesia) to a subject.

The system of the present invention may include means to provide, graphical, numeric or other forms of statistical or signal morphology related cross-linking of ECG detected arrhythmia with associated or underlying respiratory disturbance or respiratory signal. Thus arrhythmia associated with cardiac risk, as opposed to arrhythmia resulting from cardio-respiratory cross-coupling interrelationship or influence of cardio system, including the heart or ECG upon the respiratory system, including the lungs or breathing parameters (airflow; breathing effort, or various breathing path pressure changes) can be distinguished. This feature may be utilised in application of optimal therapeutic treatment to a subject under treatment.

The-system may include means for monitoring and analyzing a subject's real-time or post data acquisition ECG or pulse wave related signals including any combination of:
a) means for determining respiratory related arousals from ECG;
b) means of determining respiratory related arousals from PTT, PWA or PAT;
c) means for tracking or correlating arousals with breathing cycle;
d) means for determining whether arousal appears in co-incidence with apnea or hypopnoea termination;
e) means for distinguishing breath-related and other sources of arousal;
f) means for determination or distinguishing blood pressure influential arousals such as breath terminating arousal versus periodic leg movement or other forms of arousals;
g) means for determining from a) to d) measures or derived data appropriate diagnosis of a subject and correct counter-measure treatment for the subject;
h) means for detecting cardiac related and/or sleep related arousals while optimising countermeasure treatment for the subject including ventilatory support for eliminating SDB and for optimising sleep quality and/or efficiency;
i) means for detecting cardiac events breath by breath and/or by complete study classification and/or including monitoring sleep state, wake state;
j) means for determining whether a series of cortical related arousals could suggest onset of potentially dangerous hypertension;
k) means for determining elevated blood pressure and determining subsequent countermeasure treatment to eliminate shallow breathing or incidence of hypopnoea, which in particular may not be evident from airflow alone; and
j) means for determining if PLM related arousals would not likely warrant increase of treatment pressure.

The system may include means for detecting Sleep Disordered Breathing (SDB) including:
a) means for deriving ECG respiration from one or more ECG signals; and
b) means for comparing signal morphology to a predetermined pattern or range of pattern conditions. The comparison to a reference or pre-determined physiological SDB-related or ECG-related changes or patterns may provide a determination or classification of CSR.

The system may include means for monitoring and analyzing a subject's real-time or post data acquisition ECG signals including:
a) means to derive HRV from ECG signal; and
b) means to derive any combination values derived from HRV including: low-frequency HRV power, high-frequency HRV power, ratio of low-frequency HRV power and high-frequency HRV power as a measure of blood pressure variation; onset of hypertension or other forms of heart risk.

The system may include means for monitoring and analyzing real-time or post data acquisition ECG signals including any combination of:
a) means to augment ECG only monitoring with monitoring and analysis any combination of one or more optional channels including monitoring of physiological parameters for determining sleep state, wake state, arousal states
   (cortical or subcortical), rest state, anesthesia state, exercise state, cardiac risk, respiratory risk, and illness state, occurrence absence of REM;
b) means to simultaneously derive respiration rate and respiratory sinus arrhythmia as a measure of cardiac vagal tone and a subjects state of illness or prediction of illness onset;
c) means to derive a measure of rate of change such as integration of HRV as a means to determine breath by breath autonomic activity and subsequent markers of a subjects potential illness or mortality outcomes;
d) means to utilize correlation analysis techniques with breath by breath HRV and REM dipping or other sleep state anticipated HRV variations, to more accurately derive autonomic activity or subsequent patient cardiac risk or propensity to mortality or illness onset;
e) means to recognize states such as REM dipping and associated SDB events, respiratory and ectopic beat adjusted HRV as a marker for predicting onset of blood pressure related disorders including hypertension or preclampsia;
f) means to predetermine, one or more threshold and/or limits associated with safe or optimal operating conditions, including referencing or analyzing a data base or data bases of patient empirical or historical (medical records) data or clinical data;
g) means to determine threshold and/or limit values including derivation of a subjects safe or normal operational range of breath by breath HRV (with option of ectopic beat correction and/or respiratory coupling effects) with consideration of changes in safe operating thresholds during various sleep states (such as REM dipping) so that an alert can be generated for the subject under investigation or associated health workers, or therapeutic optimization can occur in accordance to the threshold and measured values and optimal treatment to minimize patient health risk, where the data base and/or data bases and/or patient history can relate to one individual patient, a select group of patients or a larger global or normalative data base of values;
h) heart rate;
i) HRV;
j) autonomic activity;
k) Breath by breath HRV;
l) Breath by breath integration or measure of change of HRV;
m) means for determining threshold levels associated with acquisitioned physiological data or derived analysis measures, whereby the threshold levels specify or determine normal/safe or abnormal/risk operating regions and compare currently acquisition or reviewed data or analysis for exceeding such thresholds; and
n) real-time display or link to assist in control of therapeutic treatment such as APAP, CPAP, ventilation, oxygen concentration, pace-maker, drug administration and drug perfusions.

The system may include an option of monitoring and analyzing a subject's real-time or post data acquisition ECG signals including:
a) means to augment ECG only monitoring and/or analysis with any combination of one or more optional channels including monitoring of physiological parameters including sleep state, wake state, arousal states (cortical or subcortical, or autonomic), rest state, anesthesia state, exercise state, cardiac risk, respiratory risk, and illness state; and
b) real-time display or link to assist in determination of therapeutic treatment such as APAP, CPAP, ventilation, oxygen concentration, pace-maker, drug administration, drug perfusions.

The system may include means for monitoring and analyzing a subject's real-time or post data acquisition ECG signals including:
a) means to simultaneously derive signal magnitude and phase relationship of coupling or interrelationship between cardiac function and ECG derived respiration;
b) means to determine phase relationships between consecutive heart beats and ECG-derived respiratory waveform whereby spectral analysis such as FFT may be conducted and pre-determined frequency bands of interest may be analyzed for phase relationship between R to R variability and the ECG-derived respiration signal; and
c) means to compare acquisitioned values with a global database enabling health severity or risk of monitored subject to be classified by comparing the acquisitioned values with database values, to differentiate between normal and pathological subjects with or without cardiovascular autonomic neuropathy.

The system may include means for monitoring and analyzing a subject's real-time or post data acquisitioned ECG signals including means to simultaneously derive cardiogenic oscillations and HRV, as a prediction of CSB, particularly amongst suspected or known CHF patients.

The system may include means for monitoring and analyzing a subject's real-time or post data acquisition ECG signals including:
a) ECG derived respiration and SDB combined with cross-linked (graphical, numerical, tabular, visual) autonomic measures by way of respiratory and ectopic beat corrected HRV for improved and enhanced sensitivity of state of well being and cardiac risk, onset or prediction of cardiac risk;
b) determination of a subject's sleep state;
c) respiratory and ectopic beat corrected enhanced sensitivity HRV;
d) determination of safe or normal and acceptable levels of corrected HRV to detect and pre-empt cardiac risk as measured in conjunction with anticipated variations and changes during different sleep states, where safe ranges of HRV can be determined from i) a subjects previous diagnostic study ii) empirical data studies iii) studies using mortality as an end measure of a subject's illness state;
e) means for deriving real-time and post data acquisition respiratory and ectopic beat corrected HRV enabling early indication of illness severity among patients with cardiac risk or illness presenting to an emergency department (ED) with sepsis;
f) Determination of real-time measures of HRV, as derived from L-Fnu (normalized low-frequency power), and LF/HF ratio (low-frequency/high-frequency ratio), a measure of sympathovagal balance, as a marker of illness severity and as an indicator of normal breathing, periodic breathing and CSR;
g) Real-time visual graphic, tabular or numeric display measure(s) derived from respiratory corrected HRV incorporating non-linear determination methods such as Poincare to enable quantitative display of parasympathetic nervous activity in humans;
h) Real-time determination of SDANN or SDNN and/or circadian variation of heart rate, with detection and optional alarm or therapeutic countermeasures where a pre-determined range or threshold of values mark blunting of night time heart rate define, as a means to identify sudden cardiac death survivors, determine illness severity or predict and pre-empt cardiac or respiratory risk;
i) means for scanning ECG physiological data in real-time or post data-acquisition;
j) means for determining an improved and more sensitive measure of a subject's illness by way of correcting HRV for both ectopic beats and respiration effects during a subject's sleep;
k) means for determining an improved and more sensitive measure of a subject's illness by way of correcting HRV for both ectopic beats and respiration effects during a subject's sleep, while determining the subjects sleep stage; and
l) determination and detection of reduced short terms LFP.

Control of treatment from derived parameters may include one or more of:
a) means to compare acquisitioned physiological data in real-time or post acquisition;
b) means to determine baseline or average (or other analysis means of determining past data trends) values of physiological parameters or derived analysis measures;
c) means of determining threshold levels associated with acquisitioned physiological data or derived analysis measures, whereby the threshold levels specify or determine normal/safe or abnormal/risk operating regions and compare currently acquisition or reviewed data or analysis for exceeding such thresholds where exceeding the predefined thresholds or operating regions can be determined from any combination of empirical clinical data or specific patient or patient group data;
d) violation or exceeding of threshold levels or operating regions can initiate system or user alerts, notification or changes in therapeutic treatment intervention, designed to prevent deterioration of the subject under investigation;
e) means to monitor and "store" thresholds, operation ranges, acquisitioned physiological data, derived analysis results of the physiological data to a temporary or permanent memory device which may be removable or permanently located within the acquisition system; or alternatively by way of wired or wireless connected data transfer to a remote PC, PDA or other computer or DSP based processing device; f) means to further analyse or access the "stored" data in such a way to determine or optimise diagnosis or therapeutic treatment including pacemakers, APAP, CPAP, ventilators, oxygen concentrators, drug administration or perfusion;
g) means to access the stored data in real-time or post data acquisition and compared the data with a data base of empirical data and derived analysis with various threshold and operational range definitions from any of one specific patient, a patient group or a broad population;
h) means to incorporate various sources and reference data bases of data to determine appropriate or optimal diagnosis or treatment for the subject under investigation; and
i) a subjects diagnostic data during treatment can be monitored, analysed, stored to enable derivation of measures during the subject's physiological diagnostic study, and provide a means to transfer this data to enable optimisation of therapeutic treatment for specific patient therapeutic device customisation.

The system may include means for determining heart output including any combination of:
a) heart rate;
b) HRV;
c) autonomic activity;
d) blood flow;
e) Doppler measure of blood flow such as that of utilising spectral analysis (including FFT) and determination of maximum, minimum and average blood flow characteristics associated with measured blood flow and associated blood flow turbulence;
f) Ultrasound imaging and analysis including measure of blood vessel volume or circumference, from which blood flow measures can be combined to form total heart output or heart input or cardiac output;
g) Breath by breath HRV;
h) Breath by breath integration or measure of change of HRV;
i) means of determining threshold levels associated with acquisitioned physiological data or derived analysis measures, whereby the threshold levels specify or determine normal/safe or abnormal/risk operating regions and compare currently acquisition or reviewed data or analysis for exceeding such thresholds where exceeding of predefined thresholds or operating regions can be determined from any combination of empirical clinical data or specific patient or patient group data;
j) violation or exceeding of threshold levels or operating regions can initiate system or user alerts, notification or changes in therapeutic treatment intervention, designed to prevent deterioration of the subject under investigation;
k) means to monitor and "store" thresholds, operation ranges, acquisitioned physiological data, derived analysis results of the physiological data to a temporary or permanent memory device which may be removable or permanently located within the acquisition system; or alternatively-by way of wired or wireless connected data transfer to a remote PC, PDA or other computer or DSP based processing device;
l) means to further analyse or access the "stored" data in such a way to determine or optimise diagnosis or therapeutic treatment including pacemakers, APAP, CPAP, ventilators, oxygen concentrators, drug administration or perfusion;
m) means to access the stored data in real-time or post data acquisition and compared the data with a data base of empirical data and derived analysis with various threshold and operational range definitions from either one specific patient, a patient group or a broad population;
n) means to incorporate various sources and reference data bases of data to determine appropriate or optimal diagnosis or treatment for the subject under investigation; and
o) Breath by breath risk assessment based on any combination of breath by breath HRV, breath by breath HRV change between breaths of a series of breaths or rate of change of breath by breath HRV or and subsequent alter or measure or therapeutic intervention of blood pump input output artery or volume.

The system may include means for determining an improved and more sensitive measure of a subject's illness by way of correcting HRV for both ectopic beats and respiration effects during a subject's sleep, and detection of sleep disordered breathing breath by breath classification, with graphical, numeric, tabular or visual means of cross linking changes in HRV with an associated SDB event;
means of comparing HRV measures to a global database of normal and abnormal levels, thresholds and ranges; and
means of comparing HRV measures from a subject while comparing the HRV measures to measures as retrieved from and compared to a global data base of normal and abnormal values to- detect occurrence of a subject's heart or respiratory risk or stress state, or predicting onset of same.

The system may include an option for comparing currently acquisitioned data or post acquisition data, or sequence of data, with a baseline (average or other methods) reference level derived from the monitored subject's data, for qualitative determination of short terms LFP changes that may reflect the subjects state of illness, or prediction of sudden death onset or risk of same.

The system may also include an option for comparing currently acquisitioned data or post acquisition data, or sequence of data, with health and abnormal values, thresholds or ranges of values from a global database. The global database may be derived from empirical clinical data of various illness and normal patient groups enabling thresholds and ranges of values range values. The global database may contain various categories of illness patient group (such as diabetes, SDB, heart risk and other patient groups) LFP values with classification of normal versus abnormal values and sequence of values and characteristics.

The present invention may include a system for monitoring and analyzing a subject's real-time or post data acquisition ECG signals including determination of respiratory sinus arrhythmia transfer function (RSATF) by way of estimation employing cross-power and autopower spectra.

The system may include means for monitoring and analyzing a subject's real-time or post data acquisition ECG signals including simultaneous determination of raw RR interval time series, RR consecutive difference time series and a phase portrait of the RR consecutive difference time series where phase synchronizations between these signals may be determined by evaluating relationships between respiratory signal and heart rate period in terms of power spectra and phase relations.

The system may include means for monitoring and analyzing a subject's real-time or post data acquisition ECG signals including real-time method of estimation, employing analyses of incidence of premature atrial complexes (PACs) and P-wave variability.

The system may include means for monitoring and analyzing a subject's real-time or post data acquisition ECG signals including means to derive mutual both linear and non-linear, or correlation or mutual information respectively, as a measure of coupling between heart function and respiratory function.

The system may include means for monitoring and analyzing a subject's real-time or post data acquisition ECG signals including deriving any combination of non-linear and linear analysis of HRV and ECG-derived respiration including Lyapunov exponent, CD, positive LE, noninteger CD, and nonlinearity as a measure of autonomic nervous system (ANS) processes and in measures with regard to pathophysiological disturbances and their treatment.

The system may include means for monitoring and analyzing a subject's real-time or post data acquisition ECG signals including deriving presence of RSA-like activity or subthreshold rhythmic respiratory-related activity as a likely prediction of onset of detectable SDB, respiratory disturbances or lung volume change.

The system may include means for monitoring and deriving measures from analysis of ECG signal wherein real-time or post data-acquisition analysis includes:
a) determination of time relationship between inspiration and a preceding heart beat;
b) determination of time relationship between inspiration and a following heart beat;
c) determination of phase of the cardiac cycle at which inspiration occurs;
d) determination of phases of the ventilatory cycle at which heart beats occur;
e) determination of relative phases over multiple ventilatory cycles at which heat beats occur as a measure of cardiorespiratory coupling as derived from the ECG signal; and
f) determination of synchronization between ECG-derived respiration and heart beat frequency or phase components.

The system may include means for monitoring and analyzing a subject's real-time or post data acquisition ECG signals including:
a) means to estimate ventilation by using ventilation-on-heart-rate (VE-HR) regressions established during daytime activity to estimate ventilation of a subject under real-time monitoring;
b) means to compare threshold values or ranges of threshold values from predetermined normal and abnormal health states, in order to alert subject of ventilatory risk or likely onset of same; and
c) means to measure air quality and associated ventilation-on-heart-rate (VE-HR) regressions with deterioration in air pollution as a measure of a subjects health risk or onset of same.

The system may include means for monitoring and analyzing a subject's real-time or post data acquisition ECG signals including:
a) means to determine HRV during APAP or CPAP treatment;
b) means to store HRV during treatment;
c) means to synchronize HRV with pressure augmentations;
d) means to synchronize HRV with airflow data or events such as SDB derived from such data; and
e) a visual, numerical, graphic or tabular display providing cross-linking or correlation of HRV changes, APAP or CPAP pressure changes and SDB events.

The system may include means for monitoring and analyzing a subject's real-time or post data acquisition ECG signals including:
a) means to derive from the ECG signal nocturnal paroxysmal asystole (NPA) and the number of episodes of bradycardia and pauses increased as a measure of OSAS and severity of same; and
b) a visual, numerical, graphic or tabular display providing cross-linking or correlation of HRV changes and the NPA, bradycardia and pauses, APAP or CPAP pressure changes and SDB events.

The system may include means for monitoring and analyzing a subject's real-time or post data acquisition ECG signals including:
a) determine respiratory sinus arrhythmia (RSA) low-frequency intercept, corner frequency, and roll-off in order to characterize a subject's RSA-frequency relationship during either voluntarily controlled and spontaneous breathing; and
b) a visual, numerical, graphic or tabular display providing cross-linking or correlation of HRV changes and the NPA, bradycardia and pauses, APAP or CPAP pressure changes, SDB events, (RSA) low-frequency intercept, corner frequency and roll-off.

The system may include means for monitoring and analyzing a subject's real-time or post data acquisition ECG signals, including:
a) means to determine arousals from ECG data as a measure of likely apnea termination, when detected in conjunction with apnea or potential measure of other SDB;
b) visual, numerical, graphic or tabular display providing cross-linking or correlation of HRV changes and the NPA, bradycardia and pauses, APAP or CPAP pressure changes, SDB events, (RSA) low-frequency intercept, corner frequency and roll-off;
c) real-time display or link to assist in control of therapeutic treatment such as APAP, CPAP, ventilation, oxygen concentration, pace-maker, drug administration and drug perfusions; and
d) means to monitor and "store" thresholds, operation ranges, acquisitioned physiological data, derived analysis results of the physiological data to a temporary or permanent memory device which may be removable or permanently located within the acquisition system or alternatively by way of wired or wireless connected data transfer to a remote PC, PDA or other computer or DSP based processing device.

The system may include means for monitoring and analyzing a subject's real-time or post data acquisition ECG signals, including:
a) means to determine arousal and correlation of such arousal with termination of sleep disordered breathing event such as apnea, hypopnoea or shallow breathing;
b) means to determine such arousal incidence from ECG signal by way of supplementing detection of arousal with additional signals such as pulse wave signal or cortical arousal signal;
c) means to determine arousal source by correlating detection of arousal with a breathing signal or ECG derived breathing signal in order to classify the arousal as a blood pressure change related or other source of arousal, where other sources of arousal can include movement or periodic leg movement; and
d) means to determine arousal by way of derivation from PTT analysis of ECG and pulse wave signal.

The system may include means for monitoring and analyzing a subject's real-time or post data acquisition ECG signals including:
a) means to derive EDR from a single electrode pulse and ECG;
b) means to detect and classify SDB;
c) means to detect cardiogenic oscillations by way of minute variations due to interaction between periodic breathing associated with CSR applying pressure oscillations and subsequent impedance and ECG signal variations;
d) means to detect CSR related periodic breathing or CSA related cardiogenic oscillations by way of related pressure oscillations influencing impedance and ECG signal variations from ECG signal or impedance plethysmography across ECG electrodes; and
e) means to apply or reference said measures of CSR or CSA-related cardiogenic oscillations to determination of optimal countermeasure treatment such as the control of APAP, ventilation, pacemaker or administration of gas or drugs such as oxygen or anesthesia to a subject.

The system may include means for monitoring and analyzing a subject's real-time or post data acquisition ECG signals during sleep including:
a) means to detect and classify SDB;
b) means to determine HRV and correct for ectopic beat and respiration effects to improve sensitivity of measure of autonomic or parasympathetic function during sleep;
c) means to apply or refer to the measures to determination of breath by breath autonomic or parasympathetic activity and subsequent cardiovascular risk on a breath by breath basis during a subjects sleep;
d) means to compare such measures to a global data base of normal and abnormal values, threshold and ranges of same in order to detect incidence of or onset of a subjects risk to illness or deterioration in health state; and
e) means to alert or alarm the subject being monitored or predict incidence of cardiac risk and determine a need and an optimal countermeasure treatment such as control of APAP, ventilation, pacemaker or administration of gas or drugs such as oxygen or anesthesia to a subject.

The system may include means for monitoring and analyzing a subject's real-time or post data acquisition ECG signals during sleep or wake including:
a) means to detect and classify SDB;
b) means to determine HRV and correct for ectopic beat and respiration effects to improve sensitivity of measure of autonomic or parasympathetic function during sleep;
c) means to apply or refer to measures in determination of breath by breath HRV and subsequent cardiovascular risk on a breath by breath basis during a subjects sleep;
d) means to determine HRV and correct for ectopic beat and respiration effects to improve sensitivity of measure of autonomic or parasympathetic function during sleep;
e) means to analyze a subjects sleep for breath by breath HRV and determine maximum, minimum, average, normal and abnormal HRV on a breath by breath basis;
f) an option to compare the breath by breath HRV to threshold values of normal and abnormal values based on a subjects sleep state;
g) an option to compare the breath by breath HRV to threshold values of normal and abnormal values based on a subjects age;
h) an option to compare power spectral analysis of HRV with ECG derived SDB;
i) an option to compare the breath by breath HRV to threshold values of normal and abnormal values based on a subjects health conditions such as diabetic status, hypertension risk, cardiovascular risk and genetic considerations for heart or respiratory disorders and associated risks;
j) means to compare such measures to a global database normal and abnormal values, threshold and ranges of same to detect incidence of or onset of a subjects risk to illness or health state deterioration. The global database can be used to assist in early prediction of cardiac or respiratory risk based on the degree of patient information provided. The patient information can include any combination of data including age of patient, sex, illness history, genetic disposition, sleep or wake state, coronary risk, respiratory risk, breath by breath transfer function analysis of respiratory sinus arrhythmia, subject sleep or wake state, subject position or posture; and
k) means to alert or alarm a subject being monitored or remote health worker of onset or prediction of onset of cardiac risk or need or determination of optimal countermeasure treatment such as control of APAP, ventilation, pacemaker or administration of gas or drugs such as oxygen or anesthesia to a subject.

The system may include means for monitoring and analyzing a subject's realtime or post data acquisition ECG signals during sleep or wake including:
a) means to automatically detect CSA and correlate the same with increased cardiac arrhythmias; and
b) means to produce a marker or incidence of CSA as a diagnostic measure or indicator of impaired cardiac autonomic control, increased cardiac arrhythmias and cardiac risk incidence or onset thereof.

The system may include means for monitoring and analyzing a subject's real-time or post data acquisition ECG signals during sleep or wake including:
a) means to automatically detect ECG or HRV spectral parameters a quantitative measure to augment conventional diabetes insulin based tests for diagnosis of cardiovascular autonomic neuropathy, as a measure of risk of diabetes or the onset of same;
b) an option to compare the breath by breath HRV to threshold values of normal and abnormal values based on a subjects health conditions such as diabetic status, hypertension risk, cardiovascular risk and genetic considerations for heart or respiratory disorders and associated risks;
c) means to compare such measures to a global database normal and abnormal values, threshold and ranges of same to detect incidence of or onset of a subjects risk to illness or health state deterioration. The global database can be used to assist in early prediction of cardiac or respiratory risk based on the degree of patient information provided. The patient information can include any combination of data including age of patient, sex, illness history, genetic disposition, sleep or wake state, coronary risk, respiratory risk, breath by breath transfer function analysis of respiratory sinus arrhythmia, subject sleep or wake state, subject position or posture; and
d) means to alert or alarm a subject being monitored or remote health worker of onset or prediction of onset of cardiac risk or need or determination of optimal countermeasure treatment such as control of APAP, ventilation, pacemaker or administration of gas or drugs such as oxygen or anesthesia to a subject.

The system may include means for monitoring and analyzing a subject's realtime or post data acquisition ECG signals during sleep or wake including:
a) means to automatically detect transfer function analysis of respiratory sinus arrhythmia as a quantitative measure to augment conventional diabetes insulin based tests for diagnosis of cardiovascular autonomic neuropathy;
b) an option to determine breath by breath analysis of transfer function of respiratory sinus arrhythmia as a quantitative measure;
c) an option to determine a subject sleep state as a measure that can influence transfer function analysis of respiratory sinus arrhythmia;
d) an option to determine a subject position (posture) as a measure that can influence transfer function analysis of respiratory sinus arrhythmia;
e) an option index incorporating breath by breath transfer function analysis of respiratory sinus arrhythmia with any combination of options of subject sleep or wake state, and subject position or posture; and
f) means to compare such measures to a global database or normal and abnormal values, threshold and ranges of same in order to detect incidence of or onset of a subjects risk to illness or health state deterioration.

The system may include means for monitoring and analyzing a subject's realtime or post data acquisition ECG signals during sleep or wake including:
a) means to automatically detect heart rate, respiratory rate, and strength of their interaction as a combined index or measure as a marker or measure of onset or incidence of cardiac risk, respiratory risk or health state of a subject being monitored;
b) means to compare such measures to a global database of normal and abnormal values, threshold and ranges of same to detect incidence of or onset of a subjects risk to illness or health state deterioration; and
c) means to apply or refer to the measures to determination of optimal countermeasure treatment such as control of APAP, ventilation, pacemaker or administration of gas or drugs such as oxygen or anesthesia to a subject.

The system may include means for monitoring and analyzing a subject's realtime or post data acquisition ECG signals during sleep or wake including: means including 3 or more electrodes positioned on a subject such that two separate planes of conduction are evident between each pair of electrodes, and wherein the two planes each respond to changes in thoracic and changes in abdominal breath by breath breathing effort (or lack thereof) respectively. One pair of electrodes may be positioned to measure change of impedance resulting from thoracic breathing movements, whilst a second pair of electrodes (a central electrode may be shared) may be positioned so that a change of impedance results from abdominal breathing movements.

The method may enable ECG signals to be extracted, while at the same time separate thoracic and abdominal respiratory signals may be extracted. Differentiation of abdominal and thoracic breathing in this manner with 3 or more electrodes may provide a means to determine paradoxical (out of phase) breathing associated with SDB obstructive apnea versus normal (in phase) breathing.

Breath by breath respiration or ECG analysis may include a combination of real-time on-line analysis during recording or post acquisition analysis including a combination of one or more of the following:
a) cardiovascular autonomic control system measures comprising multivariate or univariate data analysis;
b) cardiovascular autonomic control system measures based on multivariate rather than by univariate data analysis. Chaos or Deterministic chaos as a method of measurement of features of ANS;
c) measure of diminished circadian variation in HRV by way of measures of higher parasympathetic activity in patients;
(d) device and method for real-time determination of an index or measure derived from variation in HRV variables, being high-frequency (HF) component (p = 0.013) and low-frequency LF/HF ratio;
e) HRV, as derived from LFnu (normalized low-frequency power), and LF/HF (low-frequency/high-frequency) power ratio, a measure of sympathovagal balance;
f) heart rate or HRV incorporating sleep state, normal or acceptable ranges of values;
g) normal or acceptable threshold values, abnormal, irregular or suspicious ranges of values, normal or regular ranges of values, means to detect abnormal range or threshold or predict onset of same;
h) means to optimize treatment therapy as a countermeasure to prevent onset or occurrence of abnormal state or elevated cardiac, respiratory or health risk;
i) means to measure presence of reduced short-term LFP during controlled breathing as a measure or predictor of sudden death in patients with CHF that is independent of many other variables;
j) means to measure reduction in respiration and/or arousal (cortical and subcortical) as a measure or predictor of apnea and/or hypopnea - arousal can be autonomic as derived by its influence upon the ECG signal;
k) means to measure or detect presence of RSA-like activity toward end of central apnea as marker for sub-threshold rhythmic respiratory-related activity and pre-empting of onset of detectable lung volume changes or associated desaturation;
l) means of estimating pulmonary exposure and dose in air pollution epidemiology utilizing heart-rate monitoring to estimate ventilation by using ventilation-on-heart-rate (VE-HR) regressions;
m) measure of nocturnal paroxysmal asystole, episodes of bradycardia, HRV analysis, nocturnal sinusal dysfunction as a measure of parasympathetic modulation and potential incidence of OSAHS;
n) respiratory sinus arrhythmia low-frequency intercept, corner frequency, and roll-off characterizes an individual's RSA-frequency relationship during both voluntarily controlled and spontaneous breathing;
o) detection of EDR or ECG derived cardiac-induced oscillations as a measure related to relaxation of thoracic muscles during central apnea, and as such a, marker of central sleep apnea probability, as opposed to higher muscle tone during obstructive apnea, impeding cardiogenic oscillations;
p) means to apply such a marker in determination of central sleep apnea and obstructive sleep apnea EDR classification;
q) means to determine breath-by-breath HRV, or parasympathetic function. HRV includes correction for ectopic beats and the HRV maximum, minimum, and average may be determined with reference to each breath and optionally each breath and sleep state;
r) means to predict when heart rate is fixed peripheral modulation of blood pressure by respiration is clearly demonstrated;
s) cardioventilatory coupling may have a physiological role in optimizing RSA, perhaps to improve cardiopulmonary performance during sleep;
t) HRV high-frequency (HF) component and low-frequency LF/HF ratio time and frequency domain methods for heart rate variability analysis. Measurement of respiration related heart rate using Linear de-trended heart rate power spectral analysis and the Porges technique of filtered variance;
u) MPF-var technique;
v) Methods for measuring respiration-related heart rate fluctuations;
w) Removing low-frequency power from instantaneous 4Hz R-R interval signals using either a first-order linear (linear/spectral technique) or a third-order polynomial (MPF-var technique). The signals may be band-pass filtered and analyzed in both time and frequency domains. Despite the two techniques having been shown to yield substantially similar results, the MPF-var technique resulted in signal amplification at a few specific frequencies. The frequency range and effect to amplification of the MPF-var technique were found to be dependent upon polynomial size, sampling frequency, and frequency content of the signal;
x) HRV, as reflected in LFnu and the LF/HF power ratio;
y) single brief (5-minute) period of monitoring while in ED, may provide emergency physician with a readily available, noninvasive, early marker of illness severity;
z) use of a Poincare plot for quantitative display of heart rate variability allows quantitative display of parasympathetic nervous activity in humans. It has been found that that "width' of the Poincare plot is a measure of parasympathetic nervous system activity;
   aa) heart rate;
   bb) QT interval;
   cc) Heart Rate Variability;
   dd) Minimum embedding dimension (MED);
   ee) Largest Lyapunov (LLE) component;
   ff) Measures of non-linearity (NL);
   gg) Heart rate time series;
   hh) Relating heart rate variability (HRV) to change in instantaneous lung volume (ILV);
   ii) Recursive least squares (RLS) algorithm and a form of Window LMS algorithm are proposed to keep track of changes in impulse response of HRV to ILV;
   jj) fluctuation analysis of heart rate related to instantaneous lung volume presents a time domain technique for estimating transfer characteristics from fluctuations of instantaneous lung volume (ILV) to heart rate (HR);
   kk) Pre- and post-processing procedures, included pre-filtering of HR signal, pre-enhancement of high frequency content of the ILV signal, and post-filtering of estimated impulse response, together with random breathing technique, are shown to effectively reduce spurious transfer gain so as to get a stable estimate of impulse response;
   ll) Model of impulse response: Analysis of data with three components in impulse response: fast positive, delayed slow negative, and oscillatory;
   mm) premature atrial complexes (PACs) and P-wave variability;
   nn) phase synchronizations between these signals (ECG and EDR) are searched for by testing appropriate parameters of surrogate data with similar power spectra but randomly shuffled phase relations;
   oo) Mutual information (MI) analysis represents a general method to detect linear and nonlinear statistical dependencies between time series, and it can be considered as an alternative to well-known correlation analysis. Those changes and scales may be reflected by a correlation analysis. There might also be simultaneously rather large correlations, and weak dependencies, quantified by the MI. This can occur because correlation is rather different from M1; correlation describes only;
   pp) Phase relationship between heart beat periods and respiration: Identification of dynamic phase synchronizations is complicated due to changing frequency ratios of synchronized intervals, other nonstationarities, and noise. In order to overcome these problems momentary phase relations and their statistics phase synchronizations in chaotic and noisy oscillating systems could be revealed. Limitation of conventional approaches may be avoided with necessity of presetting particular frequency ratios of interest;
   qq) means to provide significant information about autonomic nervous system (ANS) processes: Lyapunov exponent (LE);
   rr) nonlinear stochastic, regular deterministic, and chaotic analysis in investigation of HRV and respiratory coupling;
   ss) Proportional Shannon entropy (H(RI-1)) of RI(-1) interval (interval between inspiration and preceding ECG R wave) as a measure of coupling, no correlation between H(RI-1) and either the fractal dimension or approximate entropy of the heart rate time series;
   tt) Cardio ventilatory coupling in atrial fibrillation comprising:
      (a) time relationship between inspiration and a preceding heart beat,
      (b) time relationship between inspiration and a following heart beat,
      (c) phase of the cardiac cycle at which inspiration occurs,
      (d) phases of the ventilatory cycle at which heart beats occur and
      (e) 'relative phases' over multiple ventilatory cycles at which heart beats
         occur;
   uu) Low-frequency intercept comprising: Respiratory sinus arrhythmia relationship markers during breathing;
   vv) Roll-off corner frequency based analysis comprising: Respiratory sinus arrhythmia relationship markers during breathing;
   ww) Measuring arousals, blood pressure and CSR in CHF patients comprising: Measure of ventilatory oscillations and arousals (can be derived from ECG via PTT or PWA (pulse wave signal available with dual ECG-Pulse-wave electrode), for example, as a marker for determination of probability of CSR;
   xx) cardiogenic oscillations on airflow signal or pulse-wave signal or ECG derived determination as a marker for central sleep apnea;
   yy) Determination of parasympathetic function during deep breathing as a measure of deep-breathing correlated or linked HRV with compensation or normal range operation consideration with older people and in individuals on cardiac medication, with left ventricular hypertrophy or ECG signs of myocardial infarction, where parasympathetic function can act as a marker inversely associated with age and left ventricular mass. This also applies to a lesser degree in healthy persons;
   zz) HR tachogram patterns derived from ambulatory ECGs for identification of sleep apnea syndrome and other sleep disturbances in patients without major autonomic dysfunction;
      aaa) measure of prevalence of central sleep apnea (CSA) in left ventricular dysfunction as a marker for impaired cardiac autonomic control and with increased cardiac arrhythmias;
      bbb) Measure of influence of respiration on human R-R interval power spectra; ccc) Combination of ECG derived SDB and arousal markers such as muscle sympathetic nerve activity (MSNA) accompanied by relatively large increases in spectral indexes of low frequency to high frequency power ratio and/or low frequency of R-R interval or HRV component (typically .04 - 0.15 Hz) and/or ratio of low frequency power to high frequency power with respiratory influences and/or corrected or compensated R-R interval;
      ddd) associate cardiac and/or CSR related arousals as a means to differentiate neural and sleep related arousals from cardiac related arousals for purposes of diagnosis and/or treatment of sleep apnea including treatment using closed loop control or open loop control or combination of closed and open loop control; eee) Spectral analysis of heart rate variability signal and respiration as a marker distinguishing normal and pathological subjects. Also applicable to diabetes patients groups to constitute a quantitative means to be added to the classical diabetic tests for diagnosis of cardiovascular autonomic neuropathy;
      fff) heart rate, R-R standard deviation (SD), R-R range (RG) and cross-correlation function (CC) computation;
      ggg) Transfer function analysis of respiratory sinus arrhythmia as a measure of autonomic function in diabetic neuropathy;
      hhh) Amplitude modulation of heart rate variability in normal full term neonates; iii) Three spectral regions of heart rate variability and identification of low frequency region below 0.02 Hz; a low frequency region from 0.02-0.20 Hz; and a high frequency region above 0.20 Hz;
      jjj) a model of cardio ventilatory coupling in order to enable a hypothetical inspiratory pacemaker to be stimulated by a signal related to cardiac action where at various levels of control the model can:
         (1) replicate all clinically described patterns of coupling;
         (2) predict variations in these described patterns and new patterns which are subsequently found in clinical time series;
         (3) simulate variations in clinically observed breathing frequency variations associated with each coupling pattern;
         (4) simulate clinically observed distribution of coupling patterns between heart rate and breathing frequency;
         (5) explain invariability of coupling below a critical heart rate/breathing frequency ratio; and
         (6) simulate changes in breathing frequency and transitions between coupling patterns from the heart rate time series of human subjects.

This model may be used to derive normal and risk values associated with cardio ventilatory coupling and causes of complex breathing rate irregularities during anesthesia, in order to pre-empt patient risk onset such as cardiac or breathing stress. Three variables in particular are modeled in order to predict or pre-empt markers of patient health state being: heart rate, intrinsic breathing frequency, and strength of their interaction;
kkk) Cardio ventilatory coupling during anesthesia. Detection enabling determination of
   1) phase coupling
   2) ratio of heart rate to ventilatory frequency
   (3) phase coupling associated with incremental changes in heart rate or ventilatory frequency, or both;
   (4) predetermined coupling patterns according to the timing relationship between the ECG R wave and start of inspiration and according to changes in the number of heart beats within each ventilatory period;
   (5) phase coupling primarily by transient changes in ventilatory period. Determination of phase coupling, in concert with respiratory sinus arrhythmia, as a measure of performance of the thoracic pump, matching cardiac filling to venous return. Determination of coupling as a marker of anesthesia relevance in conditions of impaired cardiac performance or hypovolaemia;
lll) ECG or HRV processing methods including:
   i) randomness and trend, including coherent average, cross-correlation and covariance, autocorrelation and phase-shift averaging. The relationships between commonly used frequency transforms including the Fourier series and Fourier transform for continuous time signals and extends these methods for a periodic discrete time data;
   ii) Laplace transform as an extension of the Fourier transform. The z-transform, methods based on chirp-z transform, equivalence between the time and frequency domains described in terms of Parseval's theorem and the theory of convolution, the use of the FFT for fast convolution and fast correlation for both short recordings and long recordings to be processed in sections;
   iii) Estimation of the power spectrum (PS) and coherence function (CF). PS and its estimation by means of the discrete Fourier transform considered in terms of the problem of resolution in the frequency domain. The periodogram and its variance, bias and the effects of windowing and smoothing, use of auto covariance function as a stage in power spectral estimation, and effects of windows in the autocorrelation domain, related effects of windows in the original time domain, coherence and methods by which coherence functions might be estimated; and
   iv) Mean inspiratory effort as marker of daytime sleepiness. EDR with heart rate, phase coupling, correlation of ECG phase change and transient respiratory activity.

The system may include means for monitoring and analyzing a subject's real-time or post data acquisition ECG signals and, or breathing signals during sleep, wake or anesthesia including any combination of:
a) means to determine ECG signal heart rate (heart beat rate);
b) means to determine ECG-derived respiratory signal;
c) means to determine ratio of ECG rate and respiration rate;
d) means to determine degree or measure derived from phase coupling between ECG rate and respiration rate;
e) means to determine pattern associated between start of breath inspiration and number of changes in ECG within each breath period;
f) means to determine phase coupling associated with transient changes in breath period; and
g) means to determine where the measures can provide an indication of cardiac risk associated with health risk associated with cardiac performance or hypovolaemia, and lack of cardioventilatory coupling as detected by phase and coupling diversion between cardio and respiratory signals may be a marker of impaired ventilatory or cardiac performance of the subject being monitored.

The system may include means for monitoring and analyzing a subject's real-time or post data acquisition ECG signals and/or breathing signals during sleep, wake or anesthesia including:
a) means to determine HRV;
b) means to determine respiratory sinus arrhythmia as a marker for normal cardiopulmonary performance during sleep;
c) means to control gas delivery to optimise cardioventilatory coupling of a subjects breathing and cardiac rate, where decoupling of the cardioventilatory relationship can be a sign of impaired respiratory or cardiac function and incidence or onset of respiratory or cardiac risk;
d) means to apply or refer to the measures to determination of optimal countermeasure treatment such as control of APAP, ventilation, pacemaker or administration of gas or drugs such as oxygen or anesthesia to a subject.

The system may include means for monitoring and analyzing a subject's real-time or post data acquisition ECG signals and/or breathing signals during sleep, wake or anesthesia including:
a) means to measure breath by breath respiratory effort and mean inspiratory during a subject's sleep or rest state;
b) means to determine a monitored subject's breathing effort derived from computation of average inspiratory effort; and
c) means to apply or refer to the measures to determination of optimal countermeasure treatment such as control of APAP, ventilation, pacemaker or administration of gas or drugs such as oxygen or anesthesia to a subject.

The system may include means for monitoring and analyzing a subject's real-time or post data acquisition physiological signals and/or breathing signals during sleep, wake or anesthesia including:
a) means to download in real-time or by post acquisition means a subjects acquisitioned physiological data or analysed physiological data; and
b) means to connect the data by wired or wireless means.

The system may include means for monitoring and diagnosis of a subjects respiration and Sleep Disordered Breathing (SDB) in real-time or post data acquisition including:
a) receiving a physiological ECG signal; and
b) extracting SDB parameters from the ECG signal.

The measures derived from the ECG signal may include breath-by-breath classification of sleep disordered breathing. Classification may include a determination of any one of the following categories of breathing disorders:
a) Apnea
b) Hypopnoea
c) Central sleep apnea
d) Obstructive Sleep Apnea

An implied or estimated EMG signal may be extracted from the ECG signal and an average or running average base-line EMG signal may be estimated from a predefined number of previous breaths or a pre-defined past period of time.

A subject's inferred or estimated or probability of breathing effort may be estimated from the ECG signal and this derivation may include any of the following combination of signal processing steps:
a) analyzing shape and heart rate variation of the ECG signal for inferred signs of arousal as can be associated with obstructed or partially obstructed breathing;
b) analyzing background or EMG amplitude by way of gating the QRS signal extremities or any combination of signal extremities to provide a means to amplify and assess relatively small changes and presence of EMG electrical signal activity within the larger ECG signal;
c) analyzing shape and heart rate variation of the ECG signal for inferred signs of arousal as can be associated with obstructed or partially obstructed breathing;
d) producing a m analyzing shape and heart rate variation of the ECG signal for inferred signs of arousal as can be associated with obstructed or partially obstructed breathing;
e) morphious averaged ECG signal with an option of gating (or ignoring) the ECG signal extremities so that noise is better distinguished from relatively subtle EMG signal variations which can be associated with muscle activation as is present with obstructive breathing;
f) utilizing any of the methods to derive presence of small signal oscillations or periods that can be associated with central apnea breathing oscillations, Cheyne- Stoke-Respiration or incidence of other periodic breathing; and
g) utilizing a broad bandwidth original signal typically from DC to 20 kilo Hertz bandwidth enabling higher frequency muscle and ECG signal activity and lower frequency breathing variations to extracted to as full an extent as possible.

A subject's breath classification may include any combination of the following steps:
a) determination of an airflow base-line reference (AFB) value by way of a running average or an average of a past period of time;
b) computation of each breath's signal amplitude and comparison of current breath with derived AFB;
c) determination of change in breath amplitude of the current breath when compared to the AFB;
d) determination of whether each breath is classified as an apnea, hypopnoea, normal, or movement or artefact or unknown;
e) determination whether a breath is classified as an apnea breath based on whether breath reduction from the AFB value is greater than a predefined apnea reduced breathing level (ARBL- could be for example any breath reduction greater than 80% of AFB level);
(f) determination whether a breath is classified as an hypopnoea breath based on whether breath reduction from the AFB value is greater than a predefined hypopnoea reduce breathing level (HRBL - could be for example any breath reduction between 50% and 80% of AFB level); and
g) determination whether any of the breaths is associated with or has a high probability of being associated with a cortical arousal, movement artefact or other artefact.

Each breath classification may include:
a) determination whether the breath has explicit, inferred or high probability of elevated EMG activity to infer an obstructive an obstructed or normal breath and likely level of obstructive based on EMG signal change;
b) determination whether each breath has explicit, inferred or high probability of being an apnea or hypopnoea based on derived current breath amplitude when compared to AFB value;
c) determination of central apnea breath classification based on breaths which have no inference of obstruction based on EMG activity but are recognised as an apnea and reduce airflow amplitude;
d) determination of mixed apnea breath classification based on breaths which have both a mixture breath effort or elevation of EMG activity (as caused by collapse of the upper airway palette and classified as obstructive apnea) and also have a clear indication of no EMG elevation during a reasonable portion of apnea breath indicating a central apnea (as controlled by the brain and central nervous system).

Each pair of ECG electrodes preferably generates limited energy that is below patient safety compliance maximum levels, safe amplitude and high frequency modulation (such as 100 KHz or much higher than the ECG signal of interest) between one or more ECG electrodes enabling:
a) derivation of impedance between two or more electrode contact points, where the impedance is reflective of lung function;
b) determination, from such lung function derivation, whether at any time the subject being investigated demonstrates breathing effort by way of inhalation of exhalation of the lungs and subsequent variation in real-time impedance value derivation;
c) determination based on effort of the subjects breathing and lung function whether the subject is undergoing obstructive sleep disordered breathing (lungs are attempting to suck in air and may exhibit impedance change due to physical breathing effort movement) and subsequently subtle variations are possibly exhibited within the derived inter electrode impedance monitoring; and
d) determination of breath by breath SDB classification as disclosed herein.

The system may include means for determining an optimal treatment level, which minimises or eliminates SDB. The system may include means for determining an optimal treatment level which optimises cardiac function of the subject under treatment by adjusting required treatment levels to stabilise or prevent successive arrhythmia or cardiac function which may lead to excessive blood pressure and/or states or hypertension or elevated cardiac risk. The step of adjusting may include varying the treatment level until a treatment level is reached that does not cause irregular or abnormal ECG or ECG reflective of existence, onset or potential onset of elevated cardiac risk. The step of adjusting may also include varying the treatment level until a treatment level is reached that does not cause irregular or abnormal blood-pressure or ECG and pulse-wave derived quantities or qualitative changes in blood pressure.

The system may include means for monitoring and analyzing a subject's real-time or post data acquisition ECG signals during sleep or wake including:
a) means to display both abdominal and thoracic movement ECG derived respiratory traces;
b) means to indicate acceptable placement of ECG electrodes for reliable derivation and optimal signal to noise of ECG signal;
c) means to indicate acceptable placement of ECG electrodes for reliable derivation of separately derived thoracic and abdominal respiratory signals;
d) means to prompt the system user of optimal ECG electrode placement;
e) means to graphically display a representation of ECG electrode placements with an indication of recommended ECG electrode positioning changes, if required;
f) means to detect both thoracic and abdominal respiratory traces or an indication of when ECG electrodes are placed in such a manner that the ECG signal to noise and signal quality is satisfactory;
g) means to detect both thoracic and abdominal respiratory traces or an indication of when ECG electrodes are placed in such a manner that the ECG in influence by both separate thoracic and abdominal movement axis; and
h) means to detect both thoracic and abdominal respiratory traces or an indication of when the ECG electrodes are of acceptable impedance, reflecting appropriate attachment for reliable ECG monitoring.

The system may include means for monitoring and analyzing a subject's real-time or post data acquisition ECG signals and, or breathing signals during sleep, wake or anesthesia including:
a) an ambulatory patient worn or carried device;
b) a device with capability of prompting user to enter questionnaire responses to their sleep health, including ESS or modified versions of the same or Stanford sleepiness scale or similar;
c) means to store sleepiness questionnaire results for analysis and reporting functions enabling a subjects quality of life and sleep to be assessed by patient or medical healthcare worker;
d) means to compare ESS scores of a patient with a patients history or medical recommendations and automatically notify user or a predefined location or person if any preset thresholds or recommended ranges of questionnaire values of answers suggest a health risk or fall outside recommended range;
e) means to compare specific questionnaire or states of health responses from a subject with a global database or empirical data from a group or general population of results considered normal or safe;
f) means to interface ambulatory monitoring device by wire or wireless connection to local PDA, PC, printer or other device able to present patient or healthcare worker with report printout of progressive sequence of a patient's sleep status for a single night or any series of nights;
g) means to correlate the patients sleep report status with treatment frequency and efficacy to establish graphical linkages or correlations with treatment versus a subjects sleep quality change or status;
h) means to download the sleep quality data to wire or wireless linked mobile phone for daily or routine data transfer and backup to a remote healthcare centre for further review, reporting, alerting or archiving of health records;
i) means to correlate arousal events with treatment operation and sleep quality is order to associate treatment related or induced arousals; and
j) means to compute optimised treatment control or drug delivery based on minimising arousals and maximising sleep quality.

The system may include means to enable real-time or post data computation of optimal treatment administration of APAP, CPAP, BIPAP, VPAP, ventilation, pacemaker device or oxygen concentration device including:
means to store or monitor values of ECG signal, HRV values or incidence of arrhythmia or atrial defibrillation;
means wherein the storage includes a removable or permanent memory device, and/or wire or wireless data interconnection capability and/or wire data interconnection capability;
means to compare monitored values to a pre-defined range of safe value thresholds and limits;
means to derive such thresholds and limits from a subject's diagnostic sleep or cardiac study, or healthcare-worker predefined safe thresholds or limits, or patient database derived thresholds or limits;
means to correlate real-time monitored patient parameters, with the pre-defined threshold or limit values, enabling detection of when patient parameters out of safe range; and
means to modify treatment device gas or pacemaker administration where detection of monitored parameters is out of safe operating ranges or limits.

The system may include means incorporating treatment compliance measurement including:
means to store or monitor values of ECG signal, HRV values or incidence of arrhythmia or arterial defibrillation;
means wherein said storage includes a removable or permanent memory device, and/or wired or wireless data interconnection capability;
means to transfer stored values from the treatment device to enable viewing and assessment of a subject's compliance or physiological response to the treatment.

The system may include means to determine correlation or synchrony between atrial fibrillation and sleep disordered breathing including:
means for real-time or post physiological data acquisition;
means to derive incidence or onset of (probability or likelihood of such an event) atrial fibrillation (AF) or associated symptoms or physiological event;
means to derive incidence, increased physiological stress associated with, or onset (probability or likelihood of such an event) of sleep disordered breathing (SDB) or associated physiological symptoms;
means to correlate such onset, onset probability or incidence of AF; and means to optimise therapeutic treatment as a counter measure or minimisation for either or both SDB or AF.

The system may include means to derive AF from one or more channels or physiological data and means to derive SDB from one or more channels or physiological data.

The system may include means to compute synchrony or correlation based upon signal morphology, shape and/or pattern analysis;
means to compute synchrony or correlation based upon frequency or spectral based analysis;
means to compute synchrony or correlation based upon signal phase or coherence-based analysis;
means to compute synchrony or correlation based upon amplitude and time domain based analysis; and
means to compute synchrony or correlation based upon independent component, principal component or other statistical or probability based analysis.

The system may include means to store and/or recall and/or display in real-time or post acquisition degree of, or other index or measure associated with correlation or synchrony between SDB and AF. The system may include means to store and/or recall and/or display in real-time or post acquisition AF and/or SDB raw data and/or indices or derived measures of either or both measures. The system may include means to store and/or recall and/or display in real-time or post acquisition measures associated with AF and/or SDB synchrony or correlation.

The system may include means to enable storage or recall by way of wireless data interface;
means to enable storage or recall by way of wired data interface;
means to enable storage or recall by way of removable memory card from a diagnostic device;
means to enable storage or recall by way of removable memory card from a treatment device; and
means to enable storage or recall by way of removable memory card from a combined diagnostic and treatment device.

The system may include means for adjustment or optimisation of therapeutic intervention to an individual including:
means to modify therapeutic treatment of an individual with consideration of degree of, or other index or measure associated with correlation or synchrony between SDB and AF as part of determination treatment control; and
means to modify therapeutic treatment of an individual with consideration of AF and/or SDB raw data and/or indices or derived measures of either or both measures of AF and/or SDB.

The system may include means for adjustment or optimisation of therapeutic intervention to an individual including means to modify therapeutic treatment of an individual with consideration of subjects sleep state as part of treatment control determination.

The system may include means to store and/or display and/or analyse an individuals physiological parameters including means to determine an individual's sleep state.

Sleep state determination may include any combination of:
patient airflow or pressure and/or associated analysis;
patient movement or vibration and/or associated analysis;
patient blood flow or autonomic related arousals including PTT, PWA, PAT, oximeter, ultrasonic blood flow, or pulse wave derived signals or sensors and associated analysis;
patient blood flow including PTT, PWA, PAT, oximeter, ultrasonic blood flow, or pulse wave derived signals or associated analysis; and
patient cortical arousals including EEG and/or EMG and/or movement and/or vibration derived arousals and associated analysis.

The system may include means to locally or remotely notify, alert, record or alarm personal or automated healthcare assistance. The assistance may include treatment intervention or patient assistance.

The system may include means to determine changes in blood pressure of a subject during sleep or wake and to determine from correlation of sleep state, blood pressure changes and acceptable value of change risk or prediction of natural cardiac risk such as hypertension, stroke or preeclampsia.

The system may include means to monitor blood-pressure including blood-pressure cuff based devices with manual or automatic inflation and deflation cuff capabilities with sound or pressure measures to derive associated systolic and dystolic blood pressure values; and
means to monitor blood flow and/or pressure including Doppler ultrasound based measuring devices;
means to monitor one or more patient physiological variables and derive at least 2 states of sleep from states which may include wake, stage 1, stage 2, stage 3,
rapid eye movement (REM), movement or arousal (cortical, subcortical, leg, body movement, or autonomic);
means to correlate blood pressure changes during one or more stages of sleep state with one or more thresholds or limits;
means to determine from an individual patient's empirical clinical data or patient records/history, from a group of patient's empirical clinical data or medical records/history, and/or from a global or general data base or data bases of empirical clinical data or medial record/history safe or optimal thresholds and boundaries or limits for an individual's blood pressure measures during one or more stages of sleep or during wake state;
means to alert a healthcare worker of patient where said safe limits or bounds of blood pressure are violated; and
means to modify administration of therapeutic intervention such as continuous positive air pressure (CPAP), automatic positive air pressure (APAP), Biphase positive air pressure (BIPAP), variable positive air pressure (VPAP), oxygen concentration, pacemaker, or ventilation during incidence or onset or prediction of such occurrence (blood pressure threshold or limits being exceeded).

The system of the present invention may include real-time ambulatory monitoring. Ambulatory monitoring may be provided by way of a self contained holter device. The monitoring may incorporate a capability to derive and display thoracic and abdominal ECG derived respiration traces and phase relationships, together with verification of electrode placement and guidance for a preferred connection.

Real-time derivation of EMG from ECG or superimposed on EMG may be extracted to compute breathing effort related EMG changes, such as related with OSA versus CSA.

The present invention may include a capability to record broadband ECG. Broadband may include for example DC (or 01 Hz Somte ECG high pass value) to 200 Hz or more. Broadband ECG may include a means to gate out conventional QRS pulses to enable highly sensitive measurement of residual muscle or EMG signals. Muscle signals can reflect use of abdominal or thoracic muscles as may be evident during obstructive sleep apnea, where the subject's upper airway palette typically has collapsed but neural driven autoriomic or involuntary breathing effort continues, despite the collapse of the upper airway. In contrast central sleep apnea may not be accompanied with breathing effort as breathing may be prevented due to cessation of the autonomic or involuntary neural driven mechanism.

The present system may detect relatively subtle changes in muscle activity by establishing a normal amplitude level of inter-ECG beat signal, such as by way of sampling inter-breath amplitude levels and detecting a running average level of intermediate QRS signal levels.

Respiration may be derived from one or more ECG signals. The signal morphology may, in turn, be compared to a predetermined pattern or range of pattern conditions. The pattern conditions may provide for a determination or classification of CSR.

The present invention may include means to provide, graphical, numeric or other forms of statistical or graphical cross-linking of ECG detected arrhythmia and associated or underlying respiratory disturbance or respiratory signal. Thus arrhythmia associated with cardiac risk, may be distinguished from arrhythmia resulting from cardio-cross-coupling. This function may be utilised in optimal therapeutic treatment of a subject.

In one embodiment, the system may include a holter recorder device that may be capable of storing ECG signals for a relatively long period of time (generally about 24 hours). In a preferred embodiment, a 3-lead conventional placed ECG electrode ECG-Holter with integrated (within 2 main ECG leads) resistive plethysmography and a 3-lead conventionally placed ECG electrode ECG holter with broadband frequency recorded ECG (DC to >200Hz bandwidth) may be used simultaneously. One such preferred holter recorder device is the Somte™ System manufactured by Compumedics™.

The use of a holter recorder device is desirable because it is relatively light weight and portable. This enables the holter recorder device to be easily carried by the patient during a testing period. There are also a number of devices that are capable of recording or transmitting ECG signals, such as telemetry transmitters and electrocardiograph carts. It will be readily apparent to one skilled in the art that any of these devices may be readily substituted for the disclosed holter recorder device.

In one embodiment, a recorded ECG signal may be directly transmitted to or physically loaded onto a computer-based processing system that may perform analysis as described herein. The processing system may include neural network processing methods and may provide a means to dynamically arbitrate weighting and may make use of various individual process methods, subject to factors such as reliability and quality of originating data, and behavioural and cognitive factors including a patient's state of sleep or consciousness and other measures relating to a patient's activity or behavioural state.

The system may measure broadband electrocardiogram channel, Polysomnography recordings including sleep variables (EMG, EEG, EOG and patient position) together with respiratory variables such as SaO2, airflow, upper airway resistance, respiratory effort, and breathing sounds.

SDB may be determined concurrently with performance of a cardiac study. A holter recorder device may be attached to a patient and the patient may wear the recorder device for a period of time that may include a period of sleep. The holter recorder device may record the ECG for the entire period of time, thereby enabling ECG readings to be performed during sleep. Once the study period is over, the recorded ECG may be analyzed for both cardiac disease and SDB.

As shown herein, the raw ECG signal may be processed in parallel in a number of different ways to extract cardiovascular and SDB data. The processing may include existing analysis methods, algorithms and strategies for extracting SDB-related measures from electrocardiogram signals. The analysis methods may include complex, non-linear signal source generator simulation designed to predict ECG variation, extraction of breathing signals from ECG using known impedance plethysomnography methods, heart and breathing sound analysis, ECG ectopic and other chaotic signal compensation, threshold determinations for healthy patients in contrast to presence of cardiac or breathing disorders, Cardio balistogram and other known complex signal analysis, ECG based electro-myography respiratory effort signals analysis.

The system may include a device having ambulatory or portable patient worn monitoring capability. The device may be battery operated and may include a wired or wireless interface capability. The device may be able to down load ECG derived cardiac, ventilatory or SDB data automatically without user intervention or manually by a user. The device may include means to enable remote health workers or remote scanning software to detect thresholds or ranges of measures or analysis, suggesting or indicating presence of cardiac or respiratory illness, or onset of same.

The ambulatory device may include prompts for optimal electrode placement, hot wireless to wireless override, hot battery to cable power override, battery management, multiple wireless device battery management, non-contact inductive slow-charge function or contact fast charge management function, dual trace display with phase track correlation, and hot battery replacement. The device may include displays on a head-box with capture capability including K-complex capture and freeze, spindle capture and freeze, other events capture-freeze-display, respiratory band phase validation with bargraph and traces, eye movement validation and the like, electrode stability function that analyses patients as they move for a select or predetermined period. The system may analyze continuity and consistency of impedance providing an analysis of consistency of electrode connection and stability of the connection during movement rigors. Other headbox functions or remote software functions may include artifact analysis function which may analyse signals during recording for classification according to known criteria. Artifacts may include mains, sweat artifact, EOG intrusion, excessive input electrode DC offset or change of same, unacceptable signal to noise ratio or underrated CMRR, or excessive cross-talk from other channels via a intelligent chatter comparison real-time or post recording functions, change or intermittent electrode connection, missing or poor reference and the like.

An ambulatory self-contained holter device according to the present invention may include one or more of the following features:
a) battery powered device;
b) options of wireless interconnection (blue-tooth, spread-spectrum or frequency hopping);
c) options of infra-red digital communication interface;
d) options of auto-scan and auto-detect free-band transmission;
e) option of wired connection;
f) option of battery recharge capability with hot connect and disconnect of data to local storage while monitoring including during wireless interconnect modes, with no-lost data;
g) data packet tracking with loss of data function and seamless catch up of data at later stage as required;
h) means to indicate to user remaining battery power and alert the user if there is a risk that power may be interrupted or lost, so that the user may have an option to apply a hot-wired power connect function;
i) means to monitor quality of data wireless link and alert user if there is a risk that data may be lost, so that the user may have an option to apply a hot wired data connect function;
j) means to allow the user to setup the system so that duration of study and various sample rates and channel requirements are determined and the system can compute required electrical power to complete the study, where this computation is compared to remaining battery power available for the study and the user is prompted when the study has a probability of not being completed due to insufficient remaining battery power;
k) patient worn or bedside capability including integrated within vest or fabric, wristband or watch configuration, chest or chest band attached, belt or thoracic band attached, head worn or cap integrated, arm band attached and other options;
l) integrated display capable of validating separate ECG extracted channels (including any combination of thoracic breathing effort breath by breath waveform, abdominal breathing effort breath by breath waveform, phase relationship of both said effort channels, HRV, derived pleth-wave (from ECG or additional channels), SA02 (optional channel), sleep or wake states (optional channel(s)), activity channel (rest or movements detection);
m) means to compute average phase difference between two extracted thoracic and abdominal movement respiratory traces and display via simple means such as bar graph indicating from zero to 180 degree phase shift between traces;
n) means to compute average phase difference between ECG waveforms and each breath by breath ECG respiratory movement extracted waveform and display same via simple means such as bar graphs indicating from zero to 180 degree phase shift between traces;
o) display indicator including validation of signal quality, where this can include LED displays (yes or no for quality indicators) or LCD waveform and status displays. Displays can prompt the user of correct position or change of position of sensors and electrodes. In particular the user may be provided with graphical guides and various written prompts to enable easy and clear application of ECG electrodes to facilitate continuous monitoring of change in resistive impedance attributed to thoracic breathing effort together with separate abdominal breathing effort;
p) determination of impedance between any pair of electrodes either at selected times or continuously both during data recording and other modes of system operation;
q) determination of quality of signals from each electrode in terms of background main frequency interference and signal to noise ratio at selected times or continuously both during data recording and other modes of system operation;
r) means for optional total wire-free operation including an insertable headbox wireless card and a sensor recharge technology kit;
s) means to store sensors and have them automatically recharged with clear on-board battery life remaining indication and remote alarms alerting pending status of discharge and every sensor charge status;
t) means to utilise central wireless sensor battery management so that the user can enter any combination of study length, study start, study end times and the system will prompt when data is not sufficient for the central battery management function and then prompt system user for the required entry. Once appropriate data entries are keyed in or selected by user the system may provide automatic wireless system management and guidance. Guidance may include recommendation for wired connect override and may include flashing trace LEDs and universal wire connect system for any electrode hot wire connect override to electrode wire for fast click and go battery expiration without losing any study data or troubleshooting time;
u) means to activate a remote control around one or more wireless sensors and for the remote control to issue a command that causes each wireless sensor or device to flash or indicate current battery status as a means to validate that all sensors are suitably charged or battery powered;
v) means to activate a remote control around one or more wireless sensors and for the remote control to issue a series of commands including requests of various battery duration times including a request for indication at the sensor or via a remote console as to any battery which is likely to expire within 1, 2, 3, 4 time units or any nominated time, allowing the operator to validate suitability of the multiple wireless system simply and quality at any time;
w) means to provide slow charge touchless inductive recharge for battery operated sensors or electrodes;
x) means to provide faster charge direct connect and for battery operated sensors or electrodes;
y) means to track and detect multiple sensors charging patterns and requirements and to report such charging times in a manner where all sensor charge times, and maximum charge time related to any sensor can be displayed;
z) means to prompt the user for recommended charge method (fast or slow) dependent on the users requirement for application or reuse of system and various states of charge of the sensors; and
   aa) means for the user to recognise when rechargeable batteries require replacement due to age and reduction of charge retention or reliability factors.

### Brief Description of Drawings

Preferred embodiments of the present invention will now be described with reference to the accompanying drawings wherein:-
Figure 1 shows ECG derived EMG signal waveforms during normal baseline breathing;
Figures 2a and 2b show ECG derived EMG waveforms during OSA breathing;
Figure 3 shows a block diagram overview reflecting ECG derived EMG;
Figure 4 shows a flow diagram of a system for detecting ECG-based SDB in real time or post analysis;
Figure 5 shows a flow diagram for processing an ECG signal;
Figure 6 shows a system utilizing resistive plethysmography for monitoring respiratory effort and ECG;
Figure 7 shows a sample flow diagram reflecting ECG-SDB processing;
Figure 8 shows a flow diagram reflecting analysis of HRV, ECG-SDB and countermeasures;
Figure 9 shows a flow diagram for processing ECG-derived separate signals reflecting abdominal and thoracic respiratory effort; and
Figure 10 shows features included in a self-contained holter device.

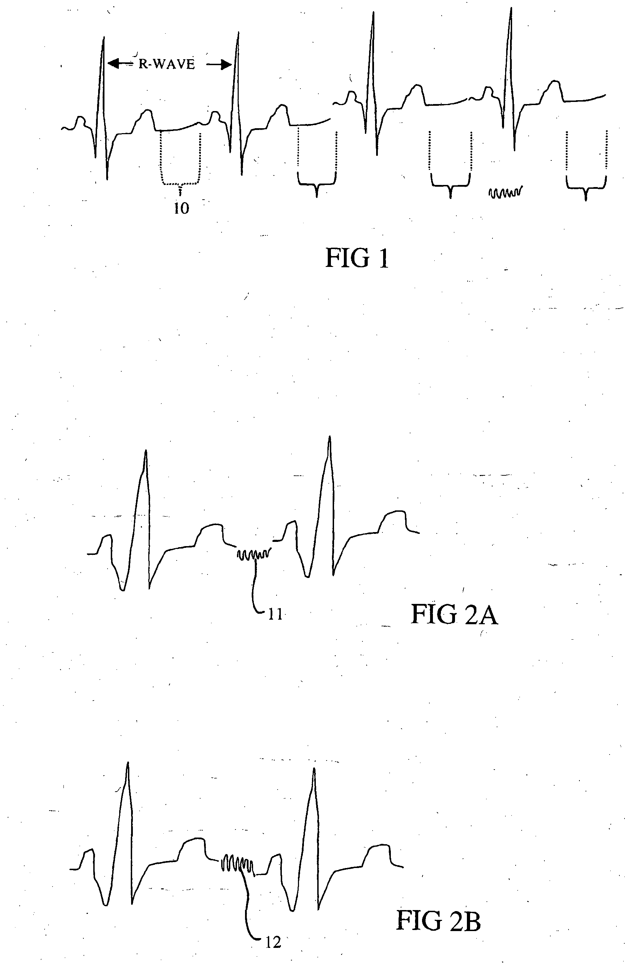

Fig. 1 shows a baseline ECG derived EMG signal during normal breathing. The Gated Inter-QRS signals 10 may enable background EMG representative of breathing muscle effort, to be amplified and measured as a marker of OSA probability. A capability to record broadband ECG being for example DC (or 01 Hz Somte ECG high pass value) to 200 Hz or more, may provide a means to gate out conventional QRS pulses and enable sensitive measurement of residual muscle signal. Muscle signal may reflect use of abdominal or thoracic muscles as may be evident during obstructive sleep apnea, where the subject's upper airway palette typically has collapsed but autonomic or involuntary breathing effort continues, despite collapse of the upper airway. In contrast central sleep apnea is not accompanied with breathing effort as breathing is prevented due to cessation of involuntary (or automatic) neural driving mechanism.

The present system may detect relatively subtle changes in muscle activity by establishing a normal amplitude level of inter-ECG beat signal, such as by way of sampling inter-breath amplitude levels and detecting a running average level of intermediate QRS signal levels.

Figs. 2a and 2b show exaggerated examples of ECG derived EMG during OSA breathing.

The residual EMG signals 11, 12 are increased when compared to the normal or average base-line EMG 10 of Fig. 1 and may suggest elevated breathing effort from either inspiratory intercostal muscles located between the ribs or the lower abdominal muscles.

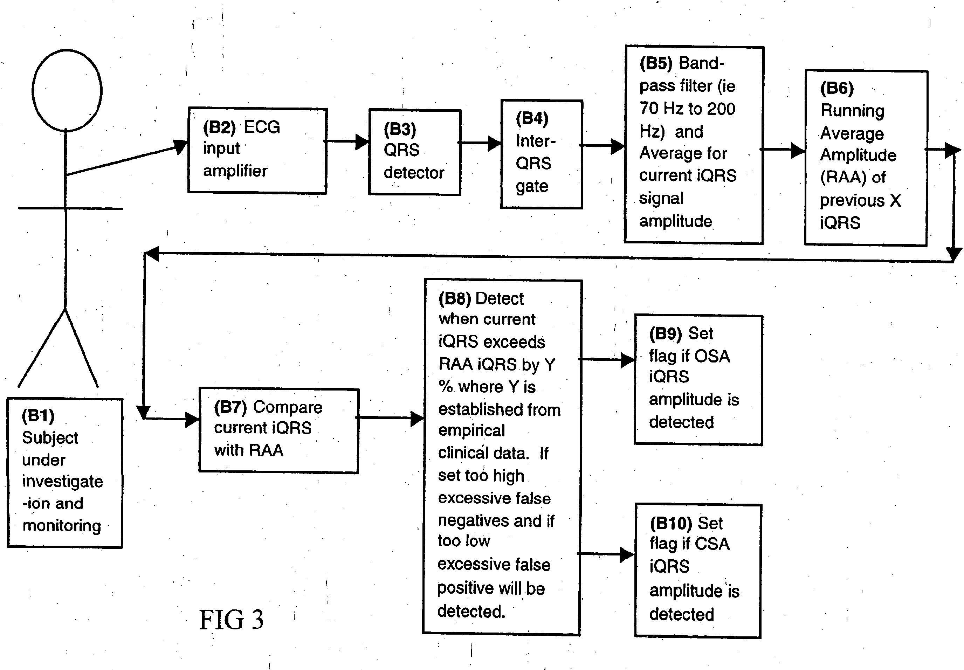

Referring to Fig. 3, block (B1) represents a subject under investigation and monitoring. Monitoring electrodes placed on subject B1 are connected to ECG input amplifier (block B2). The output of amplifier (B2) is connected to a QRS detector (block B3). The output of QRS detector (B3) is connected to Inter-QRS gate (block B4). The output of inter-QRS gate (B4) is connected to Band-pass filter (ie 70 Hz to 200 Hz) and Average for current inter-QRS (iQRS) signal amplitude detector (block B5). The output of detector (B5) is connected to block (B6) which maintains a Running Average Amplitude (RAA) of previous X iQRS. Block (B7) compares current iQRS of block (B5) with RAA of block (B6). The output of Block (B7) is connected to block (B8) which detects when current iQRS exceeds RAA iQRS by Y% where Y is established from empirical clinical data. If Y is set too high excessive false negatives will be detected and if too low excessive false positive will be detected. The output of block (B8) is connected to blocks (B9) and (B10). Block (B9) sets a flag if OSA iQRS amplitude is detected, and Block (B10) sets a flag if CSA iQRS amplitude is detected.

Further processing intelligence can be applied with the knowledge that continuous consecutive inter-QRS pulses cannot represent OSA events levels. Therefore running:

Average inter-QRS (iQRS) signal amplitude levels may be compared to running average iQRS levels. Further analysis may be applied to compare current iQRS with previous X where "X" represents for example the last 10 breaths iQRS minimal.

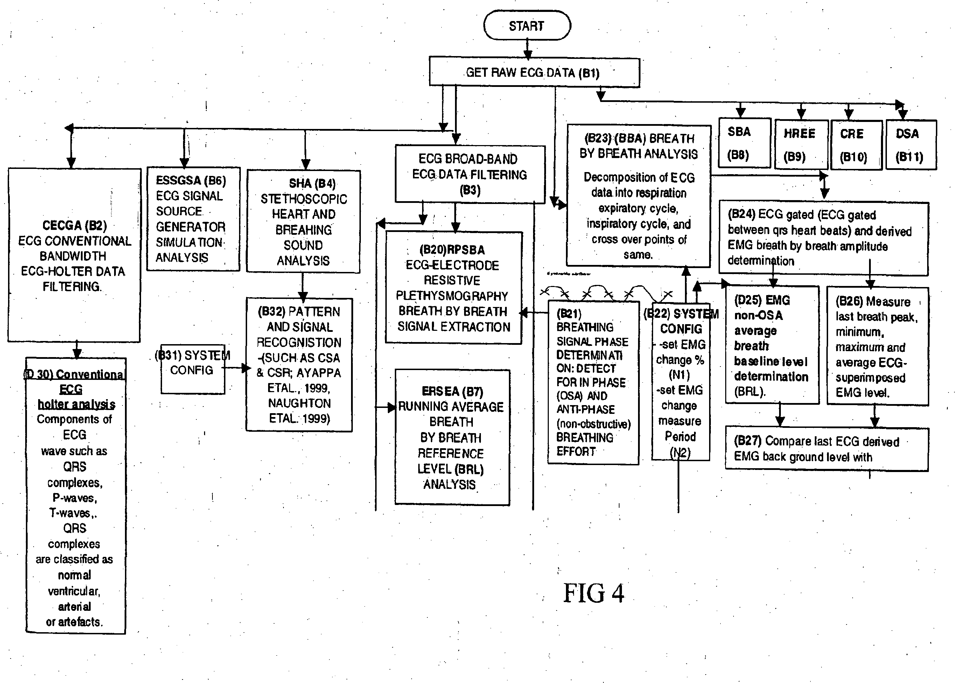

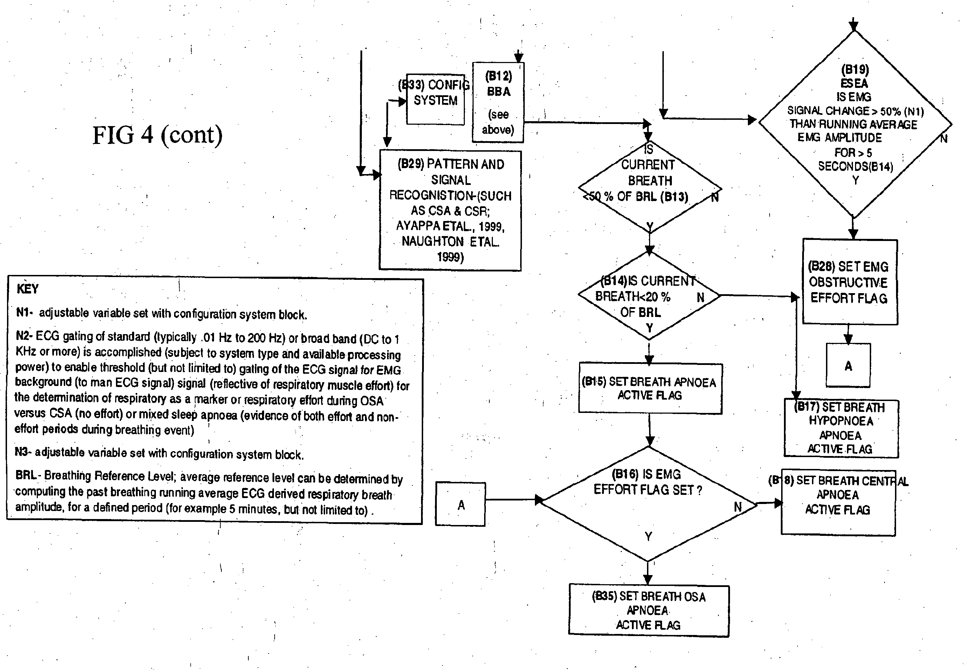

Fig. 4 shows a flow diagram of a process for detecting SDB in real-time or post analysis. The steps B1 to B35 of the process are described below.

### START

**(B1)** GET RAW ECG DATA
**(B2)** CECGA; ECG CONVENTIONAL BANDWIDTH ECG-HOLTER DATA FILTERING.
**(B30)** Conventional ECG holter analysis Components of ECG wave such as QRS complexes, P-waves, T-waves. QRS complexes are classified as normal ventricular, arterial or artefacts.
**(B6)** ESSGSA ECG SIGNAL SOURCE GENERATOR SIMULATION ANALYSIS
**(B4)** SHA STETHOSCOPIC HEART AND BREAHING SOUND ANALYSIS **(B32)** PATTERN AND SIGNAL RECOGNITION (SUCH AS CSA & CSR)
**(B31)** SYSTEM CONFIG
**(B3)** ECG BROADBAND ECG DATA FILTERING
**(B20)** RPSBA ECG-ELECTRODE RESISTIVE PLETHYSMOGRAPHY BREATH BY BREATH SIGNAL EXTRACTION
**(B21)** BREATHING SIGNAL PHASE DETERMINATION: DETECT FOR IN PHASE (OSA) AND ANTI-PHASE (non-obstructive) BREATHING EFFORT.
**(B7)** ERSEA RUNNING AVERAGE BREATH-BY-BREATH REFERENCE LEVEL (BRL) ANALYSIS.

Average breathing reference level (BRL) can be determined by computing past breathing running average ECG derived respiratory breath amplitude, for a defined period (for example 5 minutes).
**(B29)** PATTERN AND SIGNAL RECOGNITION (SUCH AS CSA & CSR)
**(B33)** CONFIG SYSTEM
**(B12)** BBA (see above)
**(B13)** IS CURRENT BREATH <50 % OF BRL?: Y OR N
**(B14)** IS CURRENT BREATH<20 % OF BRL: Y OR N
**(B17)** SET BREATH HYPOPNOEA APNEA ACTIVE FLAG
**(B15)** SET BREATH APNEA ACTIVE FLAG
**(B16)** IS EMG EFFORT FLAG SET? Y OR N
**(B18)** SET BREATH CENTRALAPNEA ACTIVE FLAG
**(B35)** SET BREATH OSA APNEA ACTIVE FLAG
**(B23) (BBA)** BREATH BY BREATH ANALYSIS Decomposition of ECG data into respiration expiratory cycle, inspiratory cycle, and cross over points of same.
**(B22)** SYSTEM CONFIG; set EMG; change % (adjustable variable set with configuration system block); set EMG change measure Period (ECG gating of standard (typically .01 Hz to 200 Hz) or broad band (DC to 1 KHz or more) is accomplished (subject to system type and available processing power) to enable threshold gating of the ECG signal for EMG background (to main ECG signal) signal (reflective of respiratory muscle effort) for determination of respiratory as a marker or respiratory effort during OSA versus CSA (no effort) or mixed sleep apnea (evidence of both effort and non-effort periods during breathing event).
**(B24)** ECG gated (ECG gated between qrs heart beats) and derived EMG breath by breath amplitude determination.
**(B25)** EMG non-OSA average breath baseline level determination (BRL).
**(B26)** Measure last breath peak, minimum, maximum and average ECG-superimposed EMG level.
**(B27)** Compare last ECG derived EMG back ground level with **(B19)** ESEA; IS EMG SIGNAL CHANGE > 50% (adjustable variable set with configuration system block) THAN RUNNING AVERAGE EMG AMPLITUDE FOR > 5 SECONDS? Y OR N
**(B28)** SET EMG OBSTRUCTIVE EFFORT FLAG
**(B8)** SBA
**(B9)** HREE
**(B10)** CRE
**(B11)** DSA

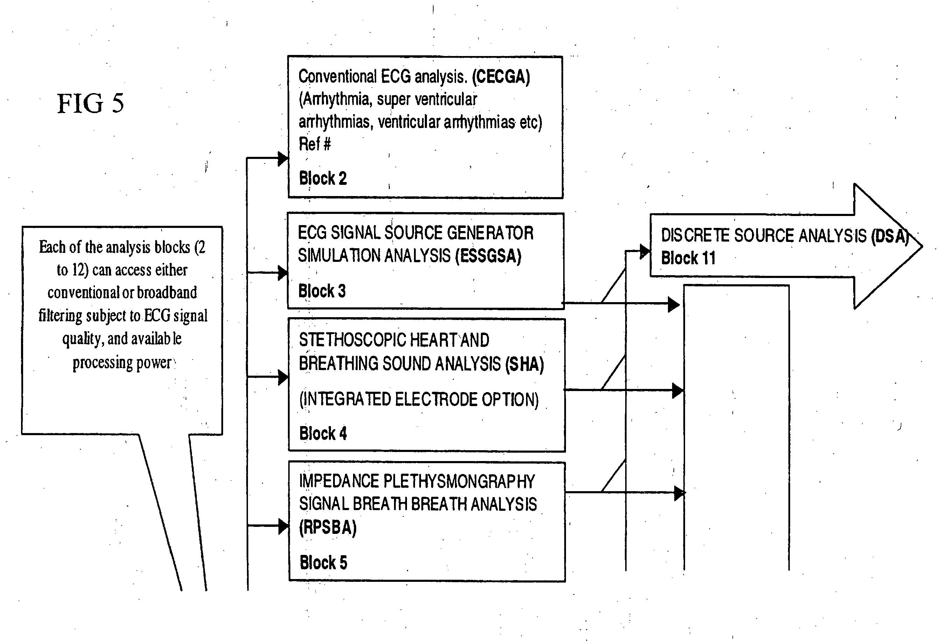

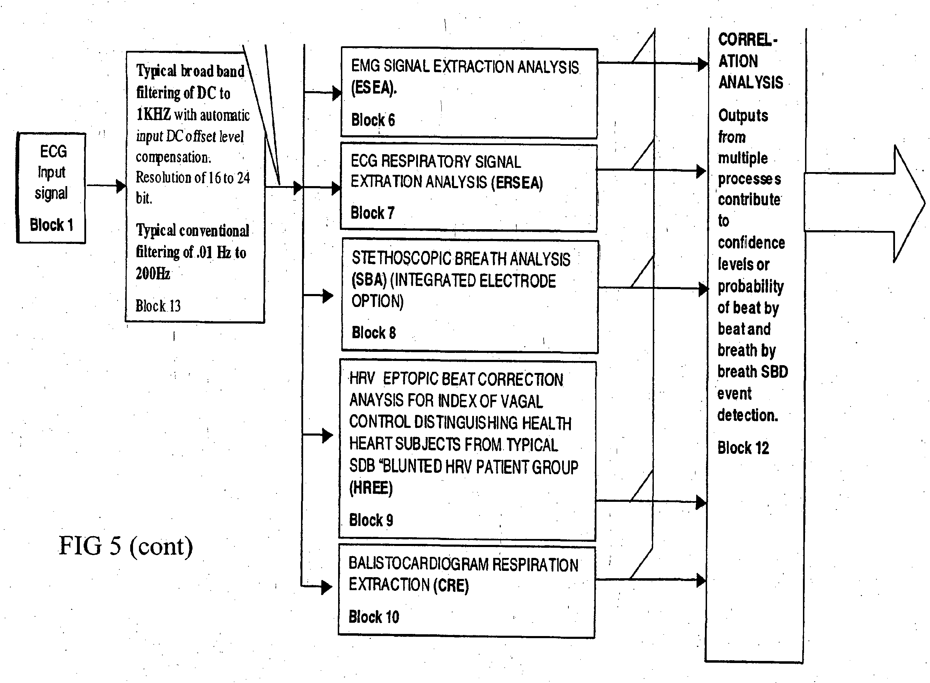

Fig. 5 is a flow diagram of one embodiment of a system for processing an ECG signal according to the present invention. A raw ECG signal (Block 1) is received and multiplexed to separate analysis modules (Blocks 2 to 12). The functions performed by the separate modules 2 to 13 are described below. In a first pathway, the raw ECG signal is filtered and a normal holter study of the ECG recordings is performed. In a second pathway, heart and breathing sounds are extracted from the ECG signal. Pattern and signal methods are used to detect for CSA and OSA. In a third pathway, the ECG undergoes broadband filtering and resistive plethysmography analysis in order to determine relative volume of each breath. The relative breath volume is used to determine apnea and hypopnea in the patient. In a further pathway, EMG signals are extracted from the raw ECG signal in order to detect obstructive breathing effort.

The ECG data is compared to historic patient data and generally accepted thresholds and norms. Theoretical simulations of individual predictive heart operation and the real time SDB models may be used to provide a broad range of ideal and real world data sets for comparison. The analysis performed by each pathway may be correlated and weighted to determine and differentiate patients with mild to severe cardiac and SDB risk.

The functions performed by Blocks 1 to 13 are described below:
**Block 1:** ECG Input signal
**Block 13:** Typical broadband filtering of DC to 1 KHZ with automatic input DC offset level compensation. Resolution of 16 to 24 bit. Typical conventional filtering of .01 Hz to 200Hz

Original broadband (DC up to 10 KHz; but typically 01 Hz to 1 KHz) ECG signal (Block 1) is presented to various analysis algorithm processes (Blocks 2 to 9). Each of the analysis modules (2 to 12) can access either conventional or broadband filtering subject to ECG signal quality, and available processing power
**Block 2:** Conventional ECG analysis. **(CECGA)** (Arrhythmia, super ventricular arrhythmias, ventricular arrhythmias etc).
**Block 3:** ECG SIGNAL SOURCE GENERATOR SIMULATION ANALYSIS **(ESSGSA)**
**Block 4:** STETHOSCOPIC HEART AND BREATHING SOUND ANALYSIS (SHA) (INTEGRATED ELECTRODE OPTION).
**Block 5:** IMPEDANCE PLETHYSMONGRAPHY SIGNAL BREATH BY BREATH ANALYSIS **(RPSBA)**
**Block 6:** EMG SIGNAL EXTRACTION ANALYSIS **(ESEA)**
**Block 7**: ECG RESPIRATORY SIGNAL EXTRATION ANALYSIS **(ERSEA)**
**Block 8:** STETHOSCOPIC BREATH ANALYSIS **(SBA)** (INTEGRATED ELECTRODE OPTION)
**Block 9:** HRV EPTOPIC BEAT CORRECTION ANAYSIS FOR INDEX OF VAGAL CONTROL DISTINGUISHING HEALTH HEART SUBJECTS FROM TYPICAL SOB "BLUNTED HRV PATIENT GROUP **(HREE).**
**Block 10:** BALISTOCARDIOGRAM RESPIRATION EXTRACTION **(CRE)**
**Block 11:** DISCRETE SOURCE ANALYSIS **(DSA)**
**Block 12:** CORREL-ATION ANALYSIS; Outputs from multiple processes contribute to confidence levels or probability of beat by beat and breath by breath SBD event detection.

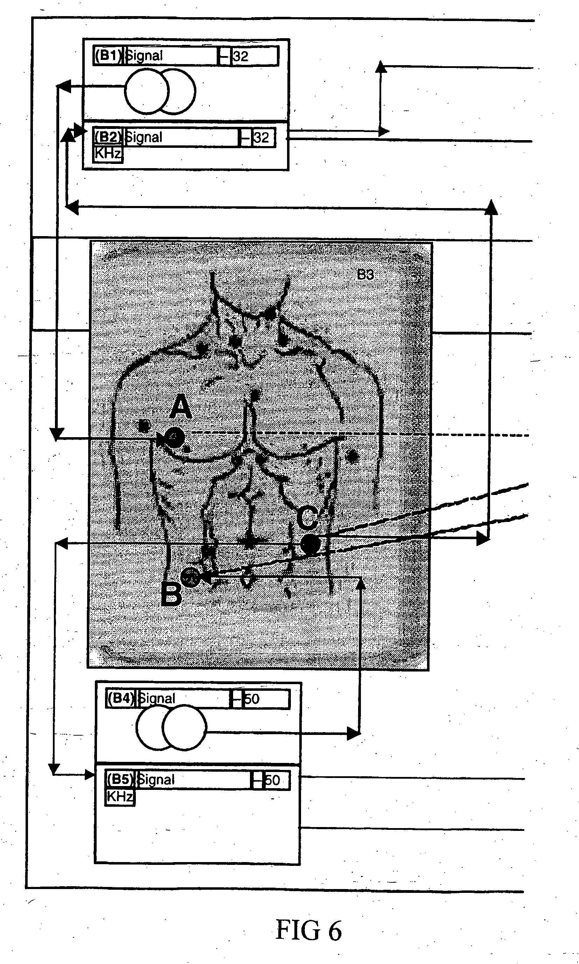

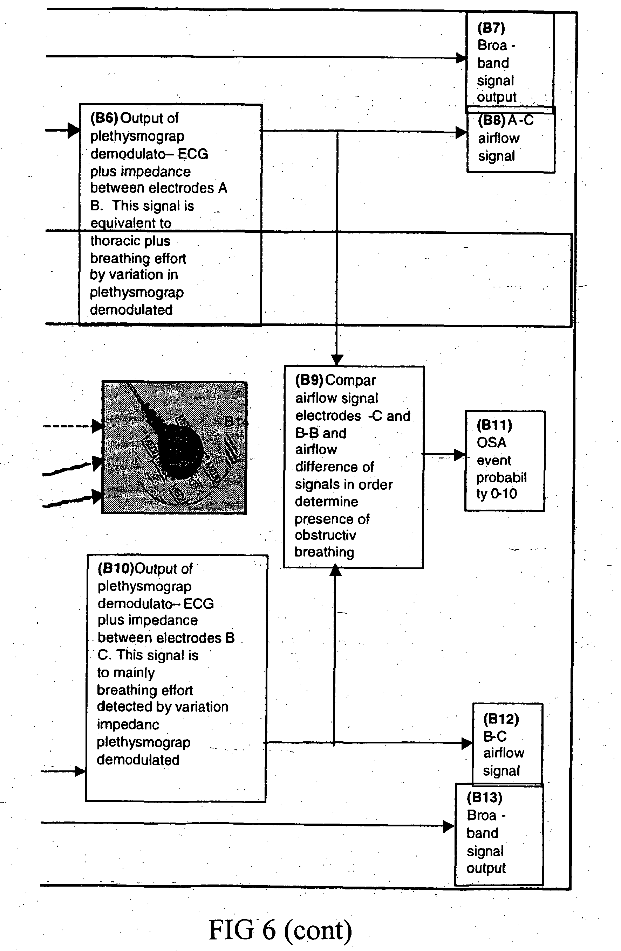

Fig. 6 shows a system utilizing resistive plethysmography including modules B1 to B13 for monitoring ECG and respiratory effort. Respiratory effort is monitored via dual frequency impedance plethysmography. The method/device enables simultaneous monitoring and analysis of SDB and cardiogram, with as few as two electrodes.

The subject B3 being monitored has 3 electrodes (A, B, C) applied to the chest/abdominal area as shown. The 3-electrode configuration may be applied for convergence of signals representing respiratory effort and cardiogram. 4 or more electrode options may also be applied, providing greater separation between signals representing thoracic and abdominal efforts and thus greater differentiation of obstructive breathing when abdominal and thoracic signals are out of phase versus non-obstructed breathing when abdominal and thoracic signals are in phase.

An AC signal (32KHz) is applied between electrodes A and C. An AC signal having a different frequency (50 KHz) is applied between electrodes B and C. As the lungs fill and empty dynamic changes in impedance across the chest cavity and between paths defined by electrodes A/C and B/C respectively, may be used to separately detect abdominal breathing effort and thoracic breathing effort. The signal between electrodes A and C represents thoracic plus abdominal breathing effort (refer B6). The signal between electrodes B and C represents abdominal breathing effort (refer B10). By comparing the two signals (amplitude) and detecting a difference in phase between the two signals, presence of obstructive breathing may be detected (refer B9).

The functions performed by modules (B1 to B13) are described below:
**(B1)** Signal generator - 32 KHz
**(B2)** Signal Demodulator - 32 KHz
**(B7)** Broadband ECG signal output
**(B8)** A-C airflow signal
**(B6)** Output of impedance plethysmography demodulator - ECG signal plus impedance variation between electrodes A and C. This signal represents mainly thoracic plus abdominal breathing effort detected by variation in impedance plethysmography demodulated signal
**(B3)** Subject being monitored and investigated
**(B4)** Signal generator - 50 KHz
**(B5)** Signal Demodulator - 50 KHz
**(B10)** Output of impedance plethysmography demodulator - ECG signal plus impedance variation between electrodes B and C. This signal represents mainly abdominal breathing effort and is detected by variation in impedance of a demodulated plethysmography signal
**(B12)** B-C airflow signal
**(B13)** Broadband ECG signal output
**(B9)** Compare airflow signals from electrodes A-C and B-C respectively and determine airflow phase difference of the two signals to determine presence of obstructive breathing.
**(B11**) OSA event probability 0-10

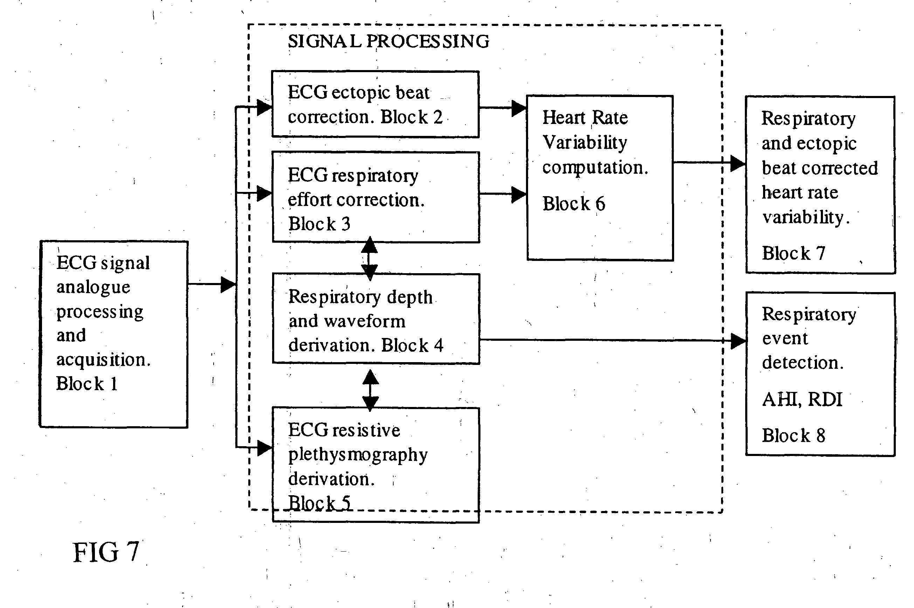

Fig. 7 shows a flow diagram including processing Blocks 1 to 8 for processing ECG-SDB signals. The functions performed by processing Blocks 1 to 8 are described below.
**Block 1:** ECG signal analogue processing and acquisition.
SIGNAL PROCESSING is performed by Blocks 2 to 6 as follows.
**Block-2:** ECG ectopic beat correction.
**Block 3:** ECG respiratory effort correction.
**Block 4:** Respiratory depth and waveform derivation.
**Block 5:** ECG resistive plethysmography derivation.
**Block 6:** Heart Rate Variability computation.
**Block 7:** Respiratory and ectopic beat corrected heart rate variability.
**Block 8:** Respiratory event detection: AHI, RDI.

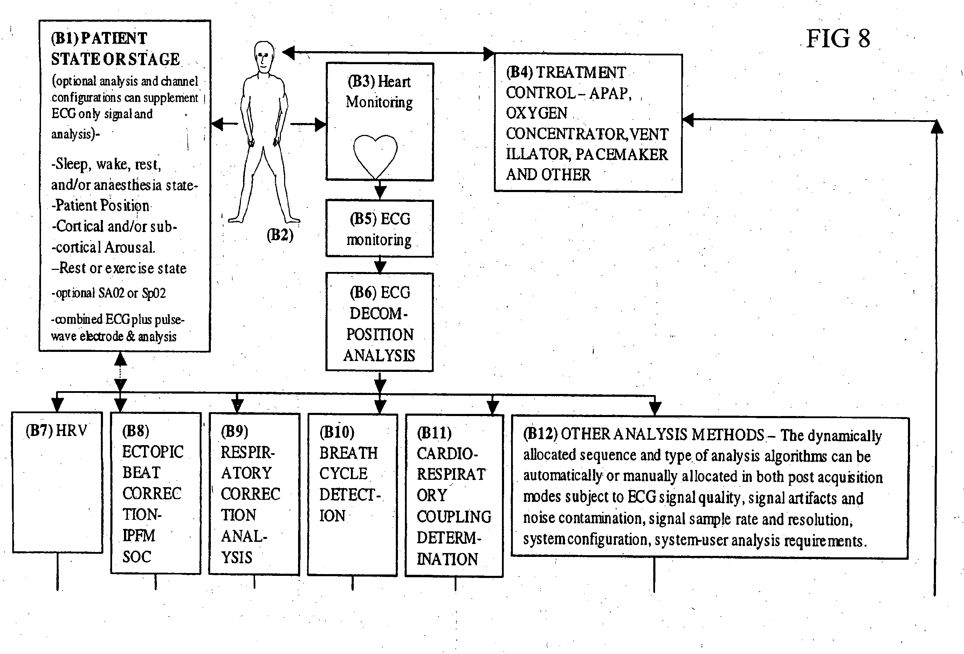

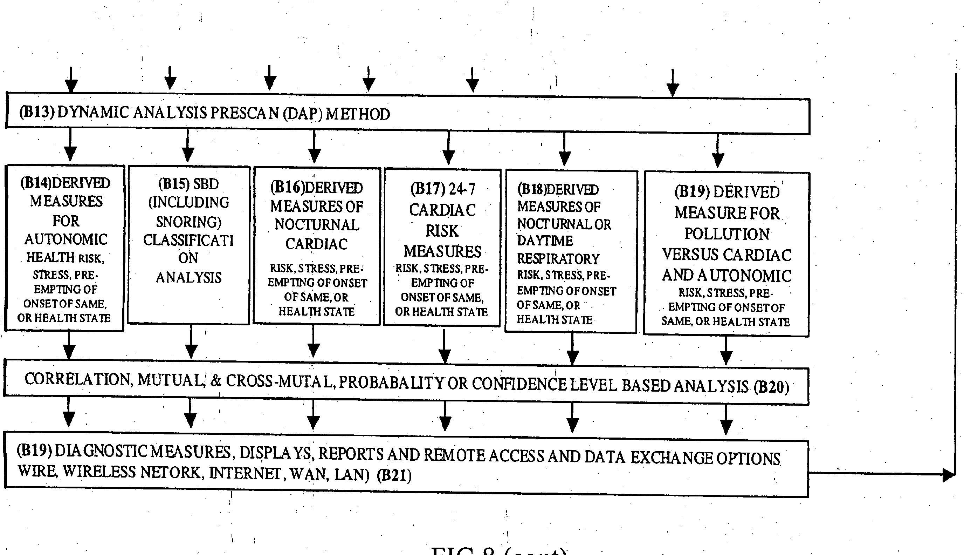

Fig. 8 shows a flow diagram including modules B1 to B21 reflecting an overview of analysis of HRV, ECG-SDB and countermeasures. The functions performed by modules B1 to B21 are described below.
**(B1)** PATIENT STATE OR STAGE (optional analysis and channel configurations can supplement ECG only signal and analysis); Sleep, wake, rest, and/or anesthesia state; Patient Position; Cortical and/or subcortical Arousal; Rest or exercise state; optional SA02 or Sp02; combined ECG plus pulse-wave electrode & analysis.
**(B2)** Subject under investigation and monitoring
**(B3)** Heart Monitoring
**(B4)** TREATMENT CONTROL - APAP, OXYGEN CONCENTRATOR, VENTILLATOR, PACEMAKER AND OTHER
**(B6)** ECG DECOMPOSITION ANALYSIS
**(B7)** HRV
(B8) ECTOPIC BEAT CORRECTION-IPFM SOC
(B9) RESPIRATORY CORRECTION ANALYSIS
**(B10)** BREATH CYCLE DETECTION WITH THORACIC AND ABDOMINAL BREATHING EFFORT DIFFERENTIATION
**(B11)** CARDIO-RESPIRATORY COUPLING DETERMINATION
(B12) OTHER ANALYSIS METHODS - The dynamically allocated sequence and type of analysis algorithms can be automatically or manually allocated in both post acquisition modes subject to ECG signal quality, signal artifacts and noise contamination, signal sample rate and resolution, system configuration, system-user analysis requirements.
**(B13)** DYNAMIC ANALYSIS PRESCAN (DAP) METHOD
**(B20)** CORRELATION, MUTUAL, & CROSS MUTAL, PROBABALITY OR CONFIDENCE LEVEL BASED ANALYSIS
(B21) DIAGNOSTIC MEASURE, DISPLAYS, REPORTS AND REMOTE ACCESS AND DATA EXCHANGE OPTIONS WIRE; WIRELESS NETORK, INTERNET, WAN, LAN

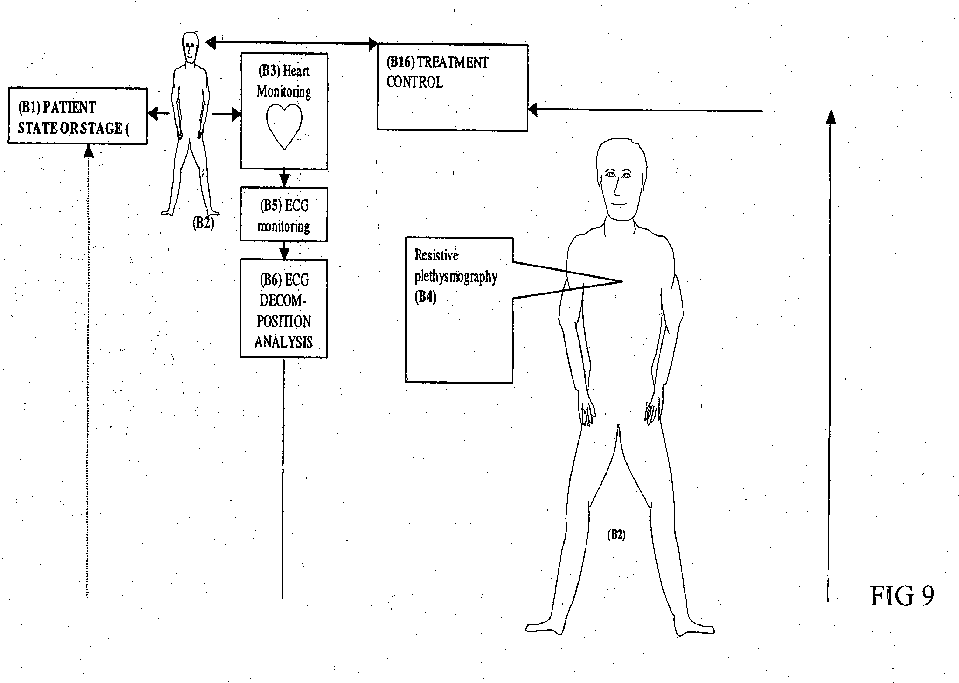

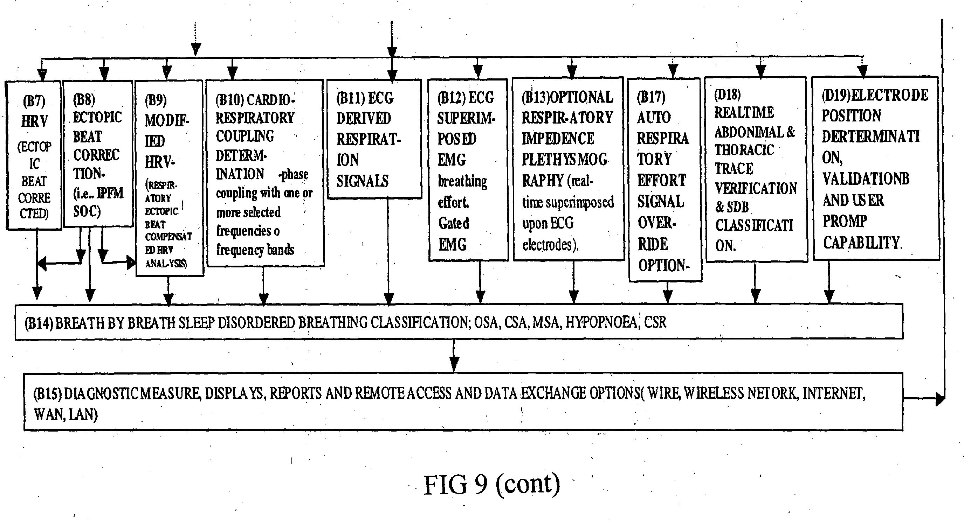

Fig. 9 shows a flow diagram of a system including modules B1 to B19 for obtaining separate signals reflecting abdominal and thoracic respiratory effort. The functions performed by analysis modules B1-B19 are described below.
**(B1)** PATIENT STATE OR STAGE (optional analysis and channel configurations can supplement ECG only analysis): Sleep, wake, rest, and/or anesthesia state; Patient Position; Cortical and/or sub cortical Arousal; Rest or exercise state; optional SA02 or Sp02; combined ECG plus pulse-wave electrode & analysis.
**(B2)** Subject under investigation and monitoring.
**(B3)** Heart Monitoring
**(B5)** ECG monitoring
**(B6)** ECG DECOMPOSITION ANALYSIS
**(B16)** TREATMENT CONTROL - APAP, OXYGEN CONCENTRATOR, VENTILLATOR, PACEMAKER AND OTHER
**(B4)** Resistive plethysmography (refer Fig. 6) produces separate and distinguishable modulation frequency between electrode A and C, from respiratory plethysmography frequency modulation between electrodes B and C. Demodulation of A to C has a greater tendency to reflect impedance changes resulting from thoracic related breathing effort, in contrast to B to C impedance changes which in contrast reflect more abdominal breathing effort changes. Differentiation of abdominal and thoracic breathing in this manner with 3 or more electrodes provides a means to determine paradoxical breathing associated with SDB obstructive apnea versus normal in phase breathing.
**(B7)** HRV (ECTOPIC BEAT CORRECTED)
**(B8)** ECTOPIC BEAT CORRECTION-(i.e. IPFM SOC)
**(B9)** MODIF-IED HRV- (RESPIR-ATORY ECTOPIC BEAT COMPENSATED HRV ANAL-YSIS)
**(B10)** CARDIO-RESPIRATORY COUPLING DETERMINATION Phase coupling with one or more selected frequencies or frequency bands
**(B11)** ECG DERIVED RESPIRATION SIGNALS- BREATH BY BREATH DETECTION WITH THORACIC AND ABDOMINAL BREATHING EFFORT DIFFERENTIATION ANALYSIS METHODS. The dynamically allocated sequence and type of analysis algorithms can be automatically or manually allocated in both post acquisition modes subject to ECG signal quality, signal artifacts and noise contamination, signal sample rate and resolution, system configuration, system-user analysis requirements.
**(B12)** ECG SUPERIMPOSED EMG breathing effort. Gated EMG
**(B13)** OPTIONAL RESPIRATORY IMPEDENCE PLETHYSMOGRAPHY (real-time superimposed upon ECG electrodes).
**(B17)** AUTO RESPIRATORY EFFORT SIGNAL OVERRIDE OPTION- The capability to supplement or displace ECG-derived respiration and SDB with abdominal and/or thoracic respiration effort signals and/or airflow signal
**(B18)** REALTIME ABDONIMAL AND THORACIC TRACE VERIFICATION & SDB CLASSIFIC-ATION. Incorporates local or remote (PDA or the like) display waveforms of ECG-derived respiration and SDB classification.
**(B19)** ELECTRODE POSITION DERTERMINATION, VALIDATION AND USER PROMPT CAPABILITY. Assist the user is ensuring that mean electrical axis produce a suitable QRS complex electrical signal appropriate for analysis, such as when the lead axis is orthogonal to the mean electrical axis, while at the same time ensuring that each electrical impedance measurement axis reflects the abdominal and thoracic movement changes respectively, to assist OSA SDB classification.
**(B14)** BREATH-BY-BREATH SLEEP DISORDERED BREATHING CLASSIFICATION; OSA, CSA, MSA, HYPOPNOEA, CSR
**(B15)** DIAGNOSTIC MEASURE, DISPLAYS, REPORTS AND REMOTE ACCESS AND DATA EXCHANGE OPTIONS(WIRED, WIRELESS NETORK, INTERNET, WAN, LAN)

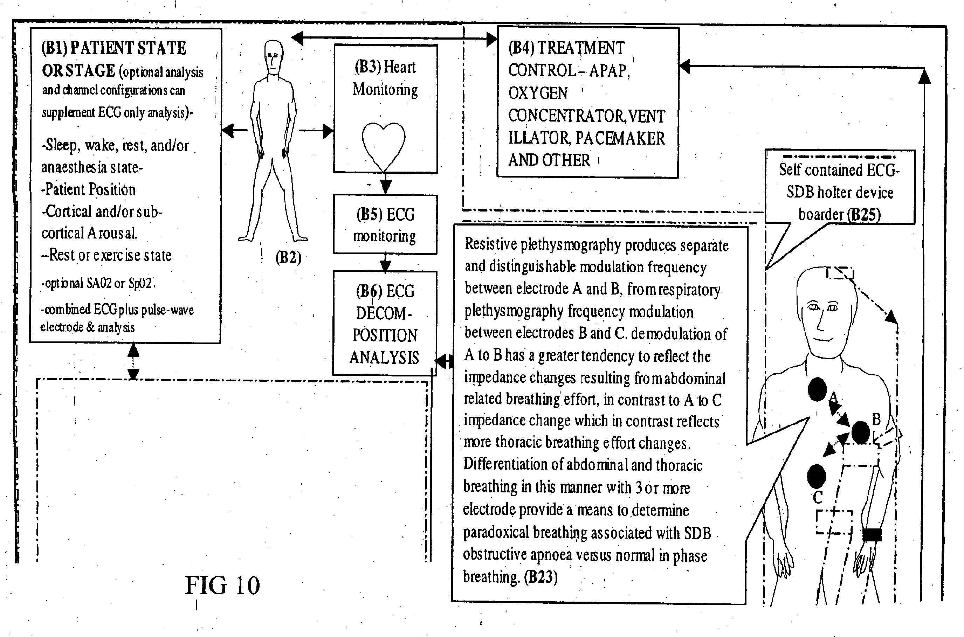

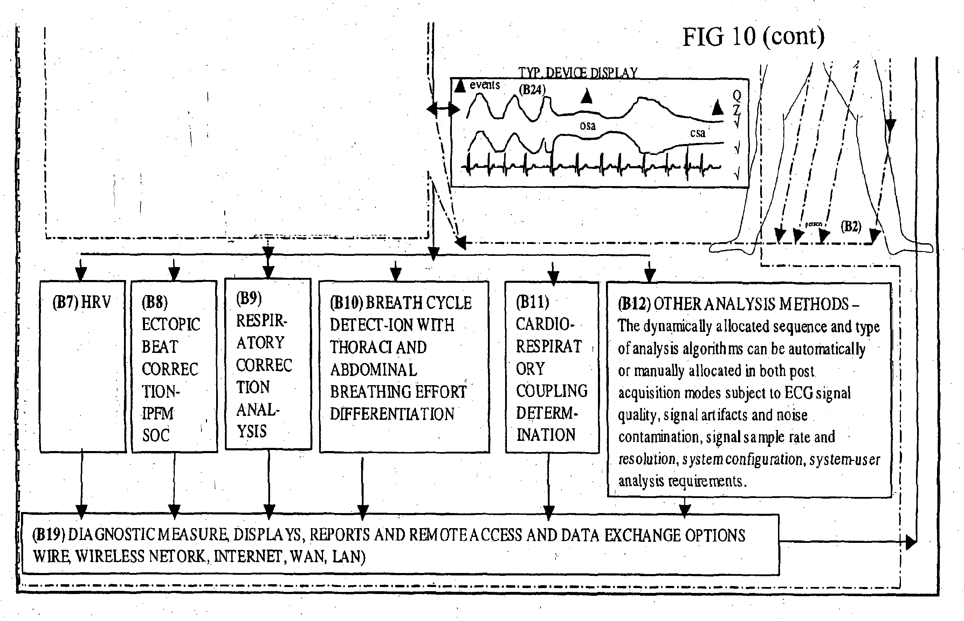

Fig. 10 shows a self contained holter device with on-board or remotely linked or wired real-time ECG-SDB signal extraction validation function. The holter device includes analysis modules B1-B3, B5-B15. The holter device is adapted to interface with treatment control module B4. The functions performed by analysis modules B1-B15 are described below.
**(B1)** PATIENT STATE OR STAGE (optional analysis and channel configurations can supplement ECG only analysis);
Sleep, wake, rest, and/or anesthesia state; Patient Position; Cortical and/or sub-cortical Arousal; Rest or exercise state; optional SA02 or Sp02; combined ECG plus pulse-wave electrode & analysis
**(B2)-** Subject under investigation and monitoring
**(B3)-** Heart Monitoring
**(B5)** ECG monitoring
**(B6)** ECG DECOMPOSITION ANALYSIS
**(B4)** TREATMENT CONTROL - APAP, OXYGEN CONCENTRATOR, VENTILATOR, PACEMAKER AND OTHER
**(B14)** Resistive plethysmography (refer Fig. 6)
**(B16)** Self contained ECG-SDB holter device border
**(B17)** SELF-CONTAINED HOLTER DEVICE

The self-contained holter device may include functions such as: wireless interconnection (blue-tooth, spread-spectrum or frequency hopping, infra-red or unique scan and auto-detect free-band transmission); wired connection option; wire connect option with battery recharge capability; guaranteed data tracking with loss less data function, patient worn or bedside capability including integration within vest or fabric, wristband or watch configuration, chest or chest band attached, abdominal or abdominal band attached, head worn or device integrated cap, arm band attachment and other options.

Options may include integrated display for validating separate ECG extracted channels including any combination of thoracic breathing effort, abdominal breathing effort, breath by breath waveform, phase relationship of both effort channels, HRV, derived pleth-wave (from ECG or additional pulse channels), SA02 (optional channel), sleep or wake states (optional channel(s)), activity channel (rest or movements detection). Display indicator includes validation of signal quality- ie. LED displays (yes or no for quality indicators) or LCD waveform and status displays. Displays may prompt the user of correct position or required change of position if abdominal respiratory and thoracic respiratory plains of monitoring cannot be distinguished or if impedance or electrodes is unsuitable, or if ECG-derived respiration is not functioning appropriately, for example.
**(B15)** TYPICAL DEVICE DISPLAY
**(B7)** HRV
**(B8)** ECTOPIC BEAT CORRECTION-IPFM SOC
**(B9)** RESPIRATORY CORRECTION ANALYSIS
**(B10)** BREATH CYCLE DETECTION WITH THORACIC AND ABDOMINAL BREATHING EFFORT DIFFERENTIATION
**(B11)** CARDIO-RESPIRATORY COUPLING DETERMINATION
**(B12)** OTHER ANALYSIS METHODS - The dynamically allocated sequence and type of analysis algorithms can be automatically or manually allocated in both post acquisition modes subject to ECG signal quality, signal artifacts and noise contamination, signal sample rate and resolution, system configuration, system-user analysis requirements.
**(B13)** DIAGNOSTIC MEASURE, DISPLAYS, REPORTS AND REMOTE ACCESS AND DATA EXCHANGE OPTIONS - WIRED, WIRELESS NETWORK, INTERNET, WAN, LAN

## Claims

1. A method of processing an electrocardiogram (ECG) signal by a system, said method comprising:
i) monitoring said ECG signal;
**characterised in that** the method further comprises:
ii) extracting from said ECG signal an inter-QRS signal, which is an EMG signal representative of breathing muscle effort, indicative of a physiological event; and
iii) analysing the inter-QRS signal to determine if the inter-QRS signal is indicative of obstructive sleep apnea (OSA) or central sleep apnea (CSA).

2. A method according to claim 1 wherein said inter-QRS signal comprises muscle signals reflecting use of abdominal or thoracic muscles as evident during obstructive sleep apnea.

3. A method according to claim 1 or 2 wherein said physiological event is derived from interaction between the heart and lungs of a subject from which the ECG signal is obtained.

4. A method according to any one of the preceding claims wherein said step of analysing the inter-QRS signal further includes the step of:
comparing said ECG signal and/or said extracted inter-QRS signal with at least one predetermined signal pattern and/or at least one threshold level and/or a 5 reference database which defines normal/safe or abnormal/risk operating regions.

5. A method according to any one of the preceding claims wherein said inter-QRS signal comprises parameters which include one or more of:
at least one of low frequency power, high frequency power, ratio of low 10 frequency to high frequency power, HRV, R to R intervals, respiratory signal, abdominal breathing effort signal, thoracic breathing effort signal and EMG breathing effort signal; and
blood pressure variation and/or onset of hypertension.

6. A method according to any one of the preceding claims wherein said ECG signal has sufficient bandwidth to enable extraction of an electromyogram (EMG) signal wherein said EMG signal provides a marker for distinguishing breathing effort characteristic of OSA classification from breathing effort characteristic of CSA classification and wherein a characteristic of said OSA classification includes at least one anti-phase signal.

7. A method according to claim 6 wherein said marker, being characteristic of OSA classification, includes an increased EMG signal indicative of breathing effort and said marker, being characteristic of CSA classification, includes a decreased EMG signal indicative of breathing effort or an absence of EMG signal indicative of breathing effort.

8. A method according to any one of the preceding claims wherein said ECG signal is provided via three ECG electrodes such that abdominal breathing effort and thoracic breathing effort may be monitored separately.

9. A method according to any one of claims 1 to 3 and 5 to 8 further 20 **characterized by** one of the following:
wherein said method is performed in real time;
wherein said method is performed breath by breath;
wherein said method is performed post offline or acquisition of said ECG signal.

10. Apparatus for detecting a physiological event in a subject (B1) from a physiological electrocardiogram (ECG) signal, including:
i) monitoring means (B1, B2) for monitoring said ECG signal;
**characterised in that** the apparatus further comprises:
ii) extracting means (B3, B4) for extracting from said ECG signal an inter-QRS signal, which is an EMG signal representative of breathing muscle effort, indicative of said event; and
iii) means (B5-B8) for analysing the inter-QRS signal to determine if the inter-QRS signal is indicative of obstructive sleep apnea (OSA) or central sleep apnea (CSA).

11. Apparatus according to claim 10 wherein said inter-QRS signal comprises muscle signals reflecting use of abdominal or thoracic muscles as evident during obstructive sleep apnea.

12. Apparatus according to claim 10 or 11 wherein said physiological event is derived from interaction between the heart and lungs of said subject (B1).

13. Apparatus according to any one of claims 10 to 12 wherein said means for analysing the inter-QRS signal includes one or a combination of:
detecting means (B8) for detecting cardiac events including incidence of arrhythmia and/or atrial fibrillation; and
classifying means for classifying sleep disordered breathing (SDB) into at least one of apnea, hypopnea, shallow breathing, CSR, CSA, OSA, MSA,
arousal, body movement, artifact, RERA, TERA and unclassified SDB wherein said classifying means further includes monitoring means (B7) for monitoring heart rate variability (HRV) and/or cardiogenic oscillations, at least for subjects diagnosed with congestive heart failure.

14. Apparatus according to any one of claims 10 to 12 wherein said means for analysing the inter-QRS signal is adapted to compare said ECG signal and/or said extracted inter-QRS signal with at least one predetermined signal pattern and/or at least one threshold level and/or a reference database which defines normal/safe or abnormal/risk operating regions.

15. Apparatus according to any one of claims 10 to 12 and 14 wherein said parameters include one or more of:
at least one of normalized low frequency power, high frequency power, ratio of low frequency to high frequency power, HRV, R to R intervals, respiratory signal, abdominal breathing effort signal, thoracic breathing effort signal and EMG breathing effort signal; and
blood pressure variation and/or onset of hypertension and/or risk or severity of heart disease.

16. Apparatus according to any one of claims 10 to 12 and 14 to 15 further including means for determining a treatment or countermeasure for said detected physiological event wherein the treatment includes one or a combination of:
APAP, CPAP, BIPAP, VPAP, ventilation, oxygen concentration, pacemaker, drug administration and/or drug perfusion.

17. Apparatus according to claim 16 wherein said means for determining is adapted to prevent arrhythmia or a condition which may lead to elevated cardiac risk including excessive blood pressure and/or a state of hypertension wherein said means for determining further includes:
means for varying said treatment to avoid an abnormal ECG signal or an ECG signal that reflects said elevated cardiac risk.

18. Apparatus according to any one of claims 10 to 17 wherein said ECG signal has sufficient bandwidth to enable extraction of an electromyogram (EMG) signal wherein said EMG signal provides a marker for distinguishing breathing effort characteristic of OSA classification from breathing effort characteristic of CSA classification and wherein a characteristic of said OSA classification includes at least one anti-phase signal.

19. Apparatus according to claim 18 wherein said marker, being characteristic of OSA classification, includes an increased EMG signal indicative of breathing effort and said marker, being characteristic of CSA classification, includes a decreased EMG signal indicative of breathing effort or an absence of EMG signal indicative of breathing effort.

20. Apparatus according to any one of claims 10 to 18 wherein said monitoring means includes three ECG electrodes attached to said subject such that
abdominal breathing effort and thoracic breathing effort may be monitored separately.

21. Apparatus according to claim 20 wherein at least one impedance path between said ECG electrodes is substantially orthogonal relative to another impedance path between said electrodes.

22. An ambulatory holter device including apparatus according to any one of claims 10 to 21.

## Patentansprüche

1. Verfahren zum Verarbeiten eines Elektrokardiogramm(EKG)-Signals mittels eines Systems, wobei dieses Verfahren umfasst:
i) das Überwachen dieses EKG-Signals,
**dadurch gekennzeichnet, dass** das Verfahren weiterhin umfasst:
ii) das Extrahieren eines inter-QRS Signals aus diesem EKG-Signal, welches ein EMG-Signal ist, repräsentativ für eine Anstrengung des Atemmuskels, als Beispiel für ein physiologisches Ereignis, und
iii) das Analysieren des inter-QRS-Signals, um zu bestimmen, ob das inter-QRS-Signal das obstruktive Schlafapnoe-Syndrom (OSA) oder das zentrale Schlafapnoe-Syndrom (CSA) anzeigt.

2. Verfahren nach Anspruch 1, bei dem dieses inter-QRS-Signal Muskelsignale einschließt, die den Gebrauch von Muskeln des Abdomens oder des Thorax während obstruktiver Schlafapnoe als nachgewiesen widerspiegeln.

3. Verfahren nach Anspruch 1 oder 2, bei dem das physiologische Ereignis abgeleitet wird aus der Interaktion zwischen Herz und Lungen eines Subjekts, von dem dieses EKG-Signal erhalten wird.

4. Verfahren nach einem der vorhergehenden Ansprüche, bei dem der Schritt des Analysierens des inter-QRS-Signals weiterhin den Schritt umfasst:
Vergleichen dieses EKG-Signals und/oder dieses extrahierten inter-QRS-Signals mit wenigstens einem vorgegebenen Signalmuster und/oder wenigstens einem Schwellenwert und/oder einer Referenz-Datenbank, die normale/sichere oder anomale/riskante Arbeitsbereiche definiert.

5. Verfahren nach einem der vorhergehenden Ansprüche, bei dem dieses inter-QRS-Signal Parameter umfasst, die einen oder mehrere einschließen aus:
wenigstens einem Niederfrequenzstrom, Hochfrequenzstrom, Verhältnis von Niedrigfrequenzstrom zu Hochfrequenzstrom, HRV, R bis R Intervall, Atemsignal, Abdomen-Atem-Versuchs-Signal, Thorax-Atem-Versuchs-Signal und EMG Atem-Versuchs-Signal, und Veränderung des Blutdrucks und/oder Beginn von Bluthochdruck.

6. Verfahren nach einem der vorhergehenden Ansprüche, bei dem dieses EKG-Signal eine ausreichende Bandbreite hat, um die Extraktion eines Elektromyogramm(EMG)-Signals zu ermöglichen, wobei dieses EMG-Signal eine Markierung liefert, um Atemversuchscharakteristika gemäß OSA-Klassifikation von Atemversuchscharakteristika gemäß CSA-Klassifikation zu unterscheiden und wobei ein Merkmal der OSA-Klassifikation wenigstens ein Anti-Phasen-Signal umfasst.

7. Verfahren nach Anspruch 6, bei dem diese Markierung, die für die OSA-Klassifizierung charakteristisch ist, ein erhöhtes EMG-Signal umfasst, welches Atemanstrengung anzeigt, und wobei diese Markierung, die für die CSA-Klassifizierung charakteristisch ist, ein verringertes EMG-Signal umfasst, das Atemanstrengung anzeigt oder eine Abwesenheit eines EMG-Signals, das Atemanstrengung anzeigt.

8. Verfahren nach einem der vorhergehenden Ansprüche, bei dem das EKG-Signal über drei EKG-Elektroden geliefert wird, derart, dass Atemanstrengung des Unterleibs und Atemanstrengung des Thorax separat angezeigt werden können.

9. Verfahren nach einem der Ansprüche 1 bis 3 und 5 bis 8, welches weiterhin **gekennzeichnet ist durch** eines der nachfolgenden Merkmale:
das Verfahren wird in Echtzeit durchgeführt,
das Verfahren wird Atemzug für Atemzug durchgeführt,
das Verfahren wird nachträglich offline oder nach Gewinnung des EKG-Signals durchgeführt.

10. Vorrichtung zur Erfassung eines physiologischen Ereignisses in einem Subjekt (B1) aus einem physiologischen Elektrokardiogramm(EKG)-Signal, umfassend:
i) Überwachungsmittel (B1, B2) für die Überwachung dieses EKG-Signals;
ii) Extraktionsmittel (B3, B4), um aus diesem EKG-Signal ein inter-QRS-Signal zu extrahieren, welches ein EMG-Signal ist, das repräsentativ ist für Anstrengungen des Atemmuskels, beispielhaft für dieses Ereignis; und
iii) Mittel (B5-B8) für das Analysieren des inter-QRS-Signals, um zu bestimmen, ob das inter-QRS-Signal obstruktive Schlafapnoe (OSA) oder zentrale Schlafapnoe (CSA) anzeigt.

11. Vorrichtung nach Anspruch 10, bei der dieses inter-QRS-Signal Muskelsignale umfasst, die den Gebrauch von Muskeln des Abdomens oder des Thorax während obstruktiver Schlafapnoe als nachgewiesen widerspiegeln.

12. Vorrichtung nach Anspruch 10 oder 11, bei der das physiologische Ereignis abgeleitet wird aus der Interaktion zwischen Herz und Lungen dieses Subjekts.

13. Vorrichtung nach einem der Ansprüche 10 bis 12, bei der diese Mittel für das Analysieren des inter-QRS-Signals eines der nachfolgenden Merkmale oder eine Kombination daraus umfassen:
Erfassungsmittel (B8) zum Erfassen von Ereignissen betreffend das Herz einschließlich dem Auftreten von Arrhythmien und/oder Herzflimmern; und
Klassifiziermittel, um schlafgestörte Atmung (SDB) zu klassifizieren in wenigstens eines ausgewählt aus Apnoe, Hypopnoe, Flachatmung, CSR, CSA, OSA, MSA, Erweckung, Körperbewegung, Artefakt, RERA, TERA und unklassifizierte SDB, wobei diese Klassifizierungsmittel weiterhin Überwachungsmittel (B7) umfassen zum Überwachen der Variabilität der Herzfrequenz (HRV) und/oder kardiogene Oszillationen, wenigstens für Subjekte, bei denen eine kongestive Herzinsuffizienz diagnostiziert wurde.

14. Vorrichtung nach einem der Ansprüche 10 bis 12, bei der diese Mittel für die Analyse des inter-QRS-Signals angepasst werden, um das EKG-Signal und/oder das extrahierte inter-QRS-Signal zu vergleichen mit wenigstens einem vorgegebenen Signalmuster und/oder wenigstens einem Schwellenwert und/oder einer Referenz-Datenbank, die normale/sichere oder anomale/riskante Arbeitsbereiche definiert.

15. Vorrichtung nach einem der Ansprüche 10 bis 12 und 14, bei der die genannten Parameter einen oder mehrere einschließen aus:
wenigstens einem normalisierten Niederfrequenzstrom, Hochfrequenzstrom, Verhältnis von Niedrigfrequenzstrom zu Hochfrequenzstrom, HRV, R bis R Intervall, Atemsignal, Abdomen-Atem-Versuchs-Signal, Thorax-Atem-Versuchs-Signal und EMG Atem-Versuchs-Signal, und Veränderung des Blutdrucks und/oder Beginn von Bluthochdruck und/oder Risiko oder Schweregrad einer Herzkrankheit.

16. Vorrichtung gemäß einem der Ansprüche 10 bis 12 und 14 bis 15, weiterhin umfassend Mittel zum Ermitteln einer Behandlung oder Gegenmaßnahme für das genannte physiologische Ereignis, wobei die Behandlung umfasst eines oder eine Kombination aus:
APAP, CPAP, BIPAP, VPAP, Beatmung, Sauerstoffkonzentration, Schrittmacher, Medikamentengabe und/oder Medikamentendurchströmung (-perfusion).

17. Vorrichtung nach Anspruch 16, bei der die Mittel für die Ermittlung geeignet sind, Arrhythmien zu verhindern oder eine Bedingung, die zu einem erhöhten kardialen Risiko führen kann, einschließlich zu hoher Blutdruck und/oder Hypertonie-Zuständen, wobei diese Mittel für die Ermittlung weiterhin umfassen:
Mittel für die Veränderung der Behandlung zum Vermeiden eines anomalen EKG-Signals oder eines EKG-Signals, welches ein erhöhtes kardiales Risiko widerspiegelt.

18. Vorrichtung nach einem der Ansprüche 10 bis 17, bei dem dieses EKG-Signal eine ausreichende Bandbreite hat, um die Extraktion eines Elektromyogramm(EMG)-Signals zu ermöglichen, wobei dieses EMG-Signal eine Markierung liefert, um Atemversuchscharakteristika gemäß OSA-Klassifikation von Atemversuchscharakteristika gemäß CSA-Klassifikation zu unterscheiden und wobei ein Merkmal der OSA-Klassifikation wenigstens ein Anti-Phasen-Signal umfasst.

19. Vorrichtung nach Anspruch 18, bei der diese Markierung, die für die OSA-Klassifizierung charakteristisch ist, ein erhöhtes EMG-Signal umfasst, welches Atemanstrengung anzeigt, und wobei diese Markierung, die für die CSA-Klassifizierung charakteristisch ist, ein verringertes EMG-Signal umfasst, das Atemanstrengung anzeigt oder eine Abwesenheit eines EMG-Signals, das Atemanstrengung anzeigt.

20. Vorrichtung nach einem der Ansprüche 10 bis 18, bei der die Überwachungsmittel drei EKG-Elektroden umfassen, die an dem Subjekt angebracht sind, derart, dass Atemanstrengung des Unterleibs und Atemanstrengung des Thorax separat angezeigt werden können.

21. Vorrichtung nach Anspruch 20, bei der wenigstens ein Impedanz-Pfad zwischen diesen EKG-Elektroden im wesentlichen orthogonal ist bezogen auf den anderen Impedanz-Pfad zwischen diesen Elektroden.

22. Ambulante Holter-EKG-Vorrichtung umfassend eine Vorrichtung gemäß einem der Ansprüche 10 bis 21.

## Revendications

1. Procédé de traitement d'un signal d'électrocardiogramme (ECG) par un système, ledit procédé comprenant :
i) surveiller ledit signal ECG ;
**caractérisé par le fait que** le procédé comprend en outre :
ii) extraire dudit signal ECG un signal inter-QRS, qui est un signal EMG représentatif d'un effort musculaire de respiration, indicatif d'un événement physiologique ; et
iii) analyser le signal inter-QRS pour déterminer si le signal inter-QRS est indicatif d'une apnée obstructive du sommeil (AOS) ou d'une apnée centrale du sommeil (ACS).

2. Procédé selon la revendication 1, dans lequel ledit signal inter-QRS comprend des signaux musculaires reflétant une utilisation de muscles abdominaux ou thoraciques comme évidente durant une apnée obstructive du sommeil.

3. Procédé selon l'une des revendications 1 ou 2, dans lequel ledit événement physiologique est déduit à partir d'une interaction entre le coeur et les poumons d'un sujet duquel le signal ECG est obtenu.

4. Procédé selon l'une quelconque des revendications précédentes, dans lequel ladite étape d'analyse du signal inter-QRS comprend en outre l'étape de :
- comparaison dudit signal ECG et/ou dudit signal inter-QRS extrait à au moins un motif de signal prédéterminé et/ou au moins un niveau de seuil et/ou une base de données de référence qui définit des régions de fonctionnement normal/sûr ou anormal/à risque.

5. Procédé selon l'une quelconque des revendications précédentes, dans lequel ledit signal inter-QRS comprend des paramètres qui comprennent l'un ou plusieurs parmi :
- au moins l'un d'une puissance basse fréquence, d'une puissance haute fréquence, d'un rapport de puissance basse fréquence à puissance haute fréquence, de VRC, d'intervalles R-R, d'un signal respiratoire, d'un signal d'effort de respiration abdominale, d'un signal d'effort de respiration thoracique et d'un signal d'effort de respiration d'EMG ; et
- une variation de pression sanguine et/ou un commencement d'hypertension.

6. Procédé selon l'une quelconque des revendications précédentes, dans lequel ledit signal ECG a une largeur de bande suffisante pour permettre une extraction d'un signal d'électromyogramme (EMG), ledit signal EMG fournissant un marqueur pour distinguer un effort de respiration caractéristique d'une classification AOS d'un effort de respiration caractéristique d'une classification ACS et une caractéristique de ladite classification AOS comprenant au moins un signal en opposition de phase.

7. Procédé selon la revendication 6, dans lequel ledit marqueur, qui est caractéristique d'une classification AOS, comprend un signal EMG accru indicatif d'un effort de respiration et ledit marqueur, qui est caractéristique d'une classification ACS, comprend un signal EMG diminué indicatif d'un effort de respiration ou une absence de signal EMG indicatif d'un effort de respiration.

8. Procédé selon l'une quelconque des revendications précédentes, dans lequel ledit signal ECG est fourni par l'intermédiaire de trois électrodes ECG de telle sorte qu'un effort de respiration abdominale et un effort de respiration thoracique peuvent être surveillés de manière séparée.

9. Procédé selon l'une quelconque des revendications 1 à 3 et 5 à 8, **caractérisé en outre par** l'un de ce qui suit :
- ledit procédé est réalisé en temps réel ;
- ledit procédé est réalisé respiration par respiration ;
- ledit procédé est réalisé après déconnexion ou acquisition dudit signal ECG.

10. Appareil pour détecter un événement physiologique chez un sujet (B1) à partir d'un signal d'électrocardiogramme physiologique (ECG), comprenant :
i) des moyens de surveillance (B1, B2) pour surveiller
ledit signal ECG ;
**caractérisé par le fait que** l'appareil comprend en outre :
ii) des moyens d'extraction (B3, B4) pour extraire dudit signal ECG un signal inter-QRS, qui est un signal EMG représentatif d'un effort musculaire de respiration, indicatif dudit événement ; et
iii) des moyens (B5-B8) pour analyser le signal inter-QRS pour déterminer si le signal inter-QRS est indicatif d'une apnée obstructive du sommeil (AOS) ou d'une apnée centrale du sommeil (ACS).

11. Appareil selon la revendication 10, dans lequel ledit signal inter-QRS comprend des signaux musculaires reflétant une utilisation de muscles abdominaux ou thoraciques comme évidente durant une apnée obstructive du sommeil.

12. Appareil selon l'une des revendications 10 ou 11, dans lequel ledit événement physiologique est déduit à partir d'une interaction entre le coeur et les poumons dudit sujet (B1).

13. Appareil selon l'une quelconque des revendications 10 à 12, dans lequel lesdits moyens pour analyser le signal inter-QRS comprennent l'un ou une combinaison de :
- des moyens de détection (B8) pour détecter des événements cardiaques comprenant un cas d'arythmie et/ou de fibrillation auriculaire ; et
- des moyens de classification pour classifier des troubles respiratoires du sommeil (TRS) en au moins l'un d'une apnée, d'une hypopnée, d'une dépression respiratoire légère, d'une respiration de Cheyne-Stokes, d'une ACS, d'une AOS, d'une apnée du sommeil mixte, d'un éveil, d'un mouvement corporel, d'un artéfact, d'un éveil lié à un effort respiratoire, d'un éveil lié à un évènement thérapeutique et de TRS non classifiés, lesdits moyens de classification comprenant en outre des moyens de surveillance (B7) pour surveiller une variabilité de la fréquence cardiaque (VRC) et/ou des oscillations cardiogènes, au moins pour des sujets diagnostiqués comme ayant une insuffisance cardiaque congestive.

14. Appareil selon l'une quelconque des revendications 10 à 12, dans lequel lesdits moyens pour analyser le signal inter-QRS sont agencés pour comparer ledit signal ECG et/ou ledit signal inter-QRS extrait à au moins un motif de signal prédéterminé et/ou au moins un niveau de seuil et/ou une base de données de référence qui définit des régions de fonctionnement normal/sûr ou anormal/à risque.

15. Appareil selon l'une quelconque des revendications 10 à 12 et 14, dans lequel lesdits paramètres comprennent l'un ou plusieurs de :
- au moins l'un d'une puissance basse fréquence normalisée, d'une puissance haute fréquence, d'un rapport de puissance basse fréquence à puissance haute fréquence, de VRC, d'intervalles R-R, d'un signal respiratoire, d'un signal d'effort de respiration abdominale, d'un signal d'effort de respiration thoracique et d'un signal d'effort de respiration d'EMG ; et
- une variation de pression sanguine et/ou un commencement d'hypertension et/ou un risque ou une gravité de cardiopathie.

16. Appareil selon l'une quelconque des revendications 10 à 12 et 14 à 15, comprenant en outre des moyens pour déterminer un traitement ou une contre-mesure pour ledit événement physiologique détecté, le traitement comprenant l'un ou une combinaison de :
- une pression positive automatique, une pression positive continue, une pression positive à deux niveaux, une ventilation en pression positive, une ventilation, une concentration en oxygène, un stimulateur cardiaque, une administration de médicament et/ou une perfusion de médicament.

17. Appareil selon la revendication 16, dans lequel lesdits moyens de détermination sont agencés pour prévenir une arythmie ou un état qui peut conduire à un risque cardiaque accru comprenant une pression sanguine excessive et/ou un état d'hypertension, lesdits moyens de détermination comprenant en outre :
- des moyens pour faire varier ledit traitement pour éviter un signal ECG anormal ou un signal ECG qui reflète ledit risque cardiaque accru.

18. Appareil selon l'une quelconque des revendications 10 à 17, dans lequel ledit signal ECG a une largeur de bande suffisante pour permettre une extraction d'un signal d'électromyogramme (EMG), ledit signal EMG fournissant un marqueur pour distinguer un effort de respiration caractéristique d'une classification AOS d'un effort de respiration caractéristique d'une classification ACS et une caractéristique de ladite classification AOS comprenant au moins un signal en opposition de phase.

19. Appareil selon la revendication 18, dans lequel ledit marqueur, qui est caractéristique d'une classification AOS, comprend un signal EMG accru indicatif d'un effort de respiration et ledit marqueur, qui est caractéristique d'une classification ACS, comprend un signal EMG diminué indicatif d'un effort de respiration ou une absence de signal EMG indicatif d'un effort de respiration.

20. Appareil selon l'une quelconque des revendications 10 à 18, dans lequel ledit moyen de surveillance comprend trois électrodes ECG attachées audit sujet de telle sorte qu'un effort de respiration abdominale et un effort de respiration thoracique peuvent être surveillés de manière séparée.

21. Appareil selon la revendication 20, dans lequel au moins un trajet d'impédance entre lesdites électrodes ECG est sensiblement orthogonal à un autre trajet d'impédance entre lesdites électrodes.

22. Dispositif Holter ambulatoire comprenant un appareil selon l'une quelconque des revendications 10 à 21.
